# EUROPEAN PATENT APPLICATION

(11) **EP 4 600 360 A1**
(43) Date of publication of application: **13.08.2025**
(21) Application number: 23874725.7
(22) Date of filing: 26.09.2023
(51) Int. Cl.: C12N 15/60, C12N 1/21, C12N 9/88, C12N 15/31, C12P 7/625

(54) **METHOD FOR MANUFACTURING COPOLYMER POLYHYDROXYALKANOIC ACID MIXTURE, AND TRANSFORMED MICROORGANISM**

(30) Priority: 03.10.2022 JP 2022159239
(71) Applicant: Kaneka Corporation, Osaka-shi, Osaka 530-8288 (JP)
(72) Inventor: ARIKAWA, Hisashi, Hyogo 676-8688 (JP); KATO, Saya, Hyogo 676-8688 (JP); MOURI, Yoshihiro, Hyogo 676-8688 (JP); SATO, Shunsuke, Hyogo 676-8688 (JP); SUGIYAMA, Hiroaki, Hyogo 676-8688 (JP); NISHIMORI, Shihomi, Hyogo 676-8688 (JP); NISHINAKA, Tomoya, Hyogo 676-8688 (JP)
(74) Representative: Vossius & Partner Patentanwälte Rechtsanwälte mbB
(86) International application number: PCT/JP2023/034999
(87) International publication number: WO 2024/075597

(57) **Abstract**

A polyhydroxyalkanoate copolymer mixture is produced by culturing a microorganism. The mixture contains: a fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and a fraction (II) that contains a polyhydroxyalkanoate having 3-hydroxybutyrate structural units and that has an average 3-hydroxyhexanoate content of 0 to 8 mol%. The weight percentage of the fraction (II) in the polyhydroxyalkanoate copolymer mixture is 45% or more.

## Description

### Technical Field

The present invention relates to a method for producing a polyhydroxyalkanoate copolymer mixture and to a transformed microorganism.

### Background Art

Polyhydroxyalkanoates (hereinafter also referred to as "PHAs") are organic polyester polymers produced by a wide variety of microorganisms. PHAs are biodegradable thermoplastic polymers and can be produced using renewable sources as raw materials. For these reasons, attempts have been made to industrially produce PHAs as environmentally conscious materials or biocompatible materials and use the produced PHAs in diverse industries.

At present, many kinds of microorganisms have been known to accumulate PHAs as energy storage substances in their cells. A typical example of the PHAs is poly-3-hydroxybutyrate (hereinafter also referred to as "P(3HB)") which is a homopolymer of 3-hydroxybutyrate (hereinafter also referred to as "3HB"). P(3HB) is a thermoplastic polymer and biologically degradable in the natural environment. This is why P(3HB) has been attracting attention as an eco-friendly plastic. However, P(3HB) is hard and brittle because of its high crystallinity, and has limited practical application. There has been a need to impart flexibility to P(3HB) in order to extend its application range.

In an attempt to increase the flexibility of PHAs, a polyhydroxyalkanoate copolymer composed of 3HB and 3-hydroxyhexanoate (hereinafter referred to as "3HH") and a method for producing the polyhydroxyalkanoate copolymer (hereinafter also referred to as "P(3HB-co-3HH)") have been studied (see Patent Literature 1 and Patent Literature 2, for example). In these reports, P(3HB-co-3HH) is produced by fermentation which uses a wild strain of *Aeromonas caviae* isolated from soil and in which the carbon source is a fatty acid such as oleic acid or palmitic acid.

A study has also been conducted in which P(3HB-co-3HH) is highly produced by using *Cupriavidus necator* as a host and using a PHA synthase derived from *Aeromonas caviae.* In the P(3HB-co-3HH) production, a vegetable oil is used as a raw material, and the 3HH content in the P(3HB-co-3HH) is increased up to about 14 mol% by introducing (R)-specific enoyl-CoA hydratase gene into *Cupriavidus necator* having an *Aeromonas caviae*-derived PHA synthase or by enhancing the expression of (R)-specific enoyl-CoA hydratase gene on the host chromosome (see Patent Literature 3, Patent Literature 4, and Non-Patent Literature 1).

There is also an example where the 3HH content in P(3HB-co-3HH) is increased to 20 mol% or more by inhibiting, in *Cupriavidus necator* having an *Aeromonas caviae*-derived PHA synthase, the expression of a gene encoding a β-ketothiolase enzyme having thiolysis activity for β-ketoacyl-CoA having six carbon atoms, i.e., β-ketohexanoyl-CoA (see Patent Literature 5).

A study about the physical properties of P(3HB-co-3HH) has also been conducted (see Non-Patent Literature 2). In this report, P(3HB-co-3HH) is produced with varying 3HH contents by fermentation in which *Aeromonas caviae* is cultured using a fatty acid having 12 or more carbon atoms as a sole carbon source. This report has revealed the following findings: the crystallinity of P(3HB-co-3HH) decreases as the 3HH content increases; thus, P(3HB-co-3HH), which is hard and brittle like P(3HB) when the 3HH content is low, becomes more flexible with increasing 3HH content; and P(3HB-co-3HH) exhibits higher flexibility than P(3HB-co-3HV) when the 3HH content is higher than a certain value. That is, the physical properties of P(3HB-co-3HH) can be varied widely from those suitable for use as a rigid polymer to those suitable for use as a soft polymer by changing the 3HH content. P(3HB-co-3HH) is therefore expected to be applicable to a wide variety of fields.

Increasing the 3HH content in P(3HB-co-3HH) provides a decrease in crystallinity and hence an improvement in flexibility. However, the increase in 3HH content tends to reduce the processability of P(3HB-co-3HH). For example, P(3HB-co-3HH) with an increased 3HH content of about 10 mol% is relatively soft, but the use of this P(3HB-co-3HH) in a process such as injection molding, film molding, blow molding, fiber spinning, extrusion foaming, or bead foaming results in low productivity due to a slow crystallization speed of the P(3HB-co-3HH).

With the goal of solving this problem, a study has been conducted in which PHAs having different monomer compositions are mixed to ensure both processability and physical properties (see Patent Literature 6).

A PHA produced by a microorganism is accumulated in the cells of the microorganism. Thus, the step of separating the PHA from the cells of the microorganism and purifying the PHA is needed to use the PHA as a plastic. The step of separating and purifying the PHA consists of disrupting the cells of the PHA-containing microorganism or solubilizing biological components other than the PHA and then collecting the PHA from the aqueous suspension resulting from the disruption or solubilization. The PHA collection is accomplished, for example, by a separation process such as centrifugation, filtration, or drying. The drying process is performed by means such as a spray dryer, fluidized-bed dryer, or drum dryer. The use of a spray dryer is preferred for the ease of the process.

Experience of the present inventors has shown that spray drying with a suitably controlled hot air temperature can elicit slight melting and fusion of the surfaces of the PHA particles to granulate the PHA into larger particles having a desired particle size. The PHA granulated in this manner is advantageous in terms of the ease of handling and the ease of transportation. The melting point of a PHA depends on the copolymerization ratio. For example, a PHA having a high 3HH content (about 9 mol% or more) has a relatively low melting point. The surfaces of particles of this PHA can be melted at a relatively low temperature to granulate the PHA into larger particles. A PHA having a low 3HH content (about 8 mol% or less) has a relatively high melting point, owing to which granulation of such a PHA requires setting a high hot air temperature. This leaves room for improvement in terms of energy saving and safety.

Patent Literature 7 teaches that spray drying of an aqueous suspension containing a PHA with a 3HH content of 0 to 8% and a PHA with a 3HH content of 9 to 25% and having a pH of 7 or less can yield a PHA powder having a large particle size even when the hot air temperature is low.

There have been reports of techniques for allowing one type of microorganism to produce different types of PHAs together (see Patent Literatures 8 to 10, for example). However, these reports fail to disclose production of any PHA having a high melting point and suitable for spray drying.

### Citation List

### Patent Literature

PTL 1: Japanese Laid-Open Patent Application Publication No. H5-93049
PTL 2: Japanese Laid-Open Patent Application Publication No. H7-265065
PTL 3: WO 2011/105379
PTL 4: WO 2015/115619
PTL 5: WO 2019/142845
PTL 6: WO 2020/195550
PTL 7: WO 2021/251049
PTL 8: WO 2015/146195
PTL 9: WO 2017/056442
PTL 10: WO 2021/206155

### Non-Patent Literature

NPL 1: H. Arikawa, K. Matsumoto, Microb.Cell.Fact., 15, p. 184 (2016)
NPL 2: Y. Doi, S. Kitamura, H. Abe, Macromolecules, 28, pp. 4822-4823 (1995)

### Summary of Invention

### Technical Problem

As described above, granulation of PHAs having a low 3HH content and a high melting point has a problem to be solved.

According to the method described in Patent Literature 7, a PHA material can be obtained which has improved granulation properties while containing a high-melting-point PHA as a main component. However, this production method involves producing two types of PHAs separately, purifying each of the produced PHAs individually to prepare an aqueous suspension of one of the PHAs and an aqueous suspension of the other PHA, then mixing the aqueous suspensions and spray-drying the resulting aqueous suspension mixture (see Examples of the literature). This method is inefficient due to the need to produce and purify two types of PHAs separately.

In view of the above circumstances, the present invention aims to provide an efficient method for producing a polyhydroxyalkanoate copolymer mixture having improved granulation properties while containing a high-melting-point PHA as a main component.

### Solution to Problem

As a result of intensive studies with the goal of solving the above problem, the present inventors have found that a polyhydroxyalkanoate copolymer mixture having improved granulation properties while containing a high-melting-point PHA as a main component can be efficiently produced by culturing a microorganism and allowing the microorganism to produce the polyhydroxyalkanoate copolymer mixture as a mixture containing two types of polyhydroxyalkanoate fractions each of which has a given composition. Based on this finding, the inventors have completed the present invention.

Specifically, the present invention relates to a method for producing a polyhydroxyalkanoate copolymer mixture, the method including the step of culturing a microorganism that produces the polyhydroxyalkanoate copolymer mixture, wherein
the polyhydroxyalkanoate copolymer mixture contains:
   a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
   a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.

The present invention also relates to a transformed microorganism that produces a polyhydroxyalkanoate copolymer mixture, the transformed microorganism having genes encoding two types of polyhydroxyalkanoate synthases differing in polymerization activity for (R)-3-hydroxyhexanoyl-CoA, wherein
the polyhydroxyalkanoate copolymer mixture contains:
   a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
   a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.

### Advantageous Effects of Invention

The present invention allows for efficient production of a polyhydroxyalkanoate copolymer mixture having improved granulation properties while containing a high-melting-point PHA as a main component. In the present invention, there is no need to individually produce each of the polyhydroxyalkanoates which are to be contained in the polyhydroxyalkanoate copolymer mixture and then mix the polyhydroxyalkanoates together, but instead the polyhydroxyalkanoate copolymer mixture can be obtained by one set of microbial culture and purification.

In addition, since the different polyhydroxyalkanoates are produced together in the cells of one microorganism, the polyhydroxyalkanoate copolymer mixture can be obtained in a well-mixed state. This makes it easy to maintain the homogeneity of the polyhydroxyalkanoate copolymer mixture in a kneading/molding process for using the mixture as an alternative plastic and renders the mixture advantageous in terms of physical properties in practical use.

In a preferred embodiment of the present invention, a powder of the polyhydroxyalkanoate copolymer mixture can be obtained by spray drying. In addition, the polyhydroxyalkanoate copolymer mixture can be granulated into powder particles with a desired particle size even when the drying temperature in the spray drying is relatively low. This is advantageous in terms of energy saving and safety.

### Description of Embodiments

Hereinafter, an embodiment of the present invention will be described. The present invention is not limited to the embodiment described below.

The present invention is directed to a method for producing a PHA copolymer mixture, the method including the step of culturing a microorganism that produces the PHA copolymer mixture.

### (PHA Copolymer Mixture)

The PHA copolymer mixture is composed of: a PHA fraction (I) that contains a PHA copolymer having 3HB structural units and 3HH structural units and that has an average 3HH content of 9 to less than 20 mol%; and a PHA fraction (II) that contains a PHA having 3HB structural units and that has an average 3HH content of 0 to 8 mol%. The PHA fraction (I) can be separately collected from the PHA copolymer mixture by MIBK fractionation described later to assay the average 3HH content of the PHA fraction (I). The average 3HH content of the PHA fraction (II) of the PHA copolymer mixture can be assayed through melting point measurement using a differential scanning calorimeter (DSC).

The PHA fraction (I) is a fraction containing a PHA copolymer having at least 3HB structural units and 3HH structural units. The PHA fraction (I) may contain a PHA having hydroxyalkanoate structural units other than 3HB and 3HH structural units. Preferably, the PHA fraction (I) is a fraction containing a PHA copolymer having only 3HB and 3HH structural units and having no hydroxyalkanoate structural units other than 3HB and 3HH structural units. That is, the PHA fraction (I) is preferably a fraction that contains P(3HB-co-3HH).

Examples of hydroxyalkanoate structural units other than 3HB and 3HH structural units include, but are not limited to, structural units derived from hydroxyalkanoates such as 3-hydroxypropionate, 3-hydroxyvalerate ("3HV"), 3-hydroxyalkanoates having 7 to 16 carbon atoms, 2-hydroxyalkanoates having 4 to 16 carbon atoms, 4-hydroxyalkanoates (such as 4-hydroxybutyrate), 5-hydroxyalkanoates, 6-hydroxyalkanoates (such as 6-hydroxyhexanoate), and lactic acid.

The average 3HH content in the PHA fraction (I) is 9 mol% or more and preferably 10 mol% or more. The PHA fraction (I) with such a high average 3HH content has a low melting temperature. Thus, the PHA fraction (I) can enter a molten state during spray drying of the PHA copolymer mixture even when the drying temperature is relatively low. It is inferred that the PHA fraction (I) in a molten state functions as a binder to aggregate particles of the non-molten PHA fraction (II) together and therefore that a PHA powder having a large particle size can be obtained even when the drying temperature is relatively low.

The average 3HH content in the PHA fraction (I) is less than 20 mol%, preferably 19.9 mol% or less, more preferably 19 mol% or less, and even more preferably 18 mol% or less.

The PHA fraction (II) is a fraction containing a PHA having 3HB structural units. The PHA contained in the PHA fraction (II) may be a homopolymer having only 3HB structural units or may be a PHA copolymer having 3HB structural units and other hydroxyalkanoate structural units. The PHA copolymer is preferably a PHA copolymer having 3HB structural units and 3HV and/or 3HH structural units, more preferably a PHA copolymer having 3HB structural units and 3HH structural units, and even more preferably a PHA copolymer having only 3HB structural units and 3HH structural units, namely, P(3HB-co-3HH).

The average 3HH content in the PHA fraction (II) is from 0 to 8 mol%. When the average 3HH content in the PHA fraction (II) is in this range, the resulting PHA copolymer mixture can be formed into a molded article having a good balance of flexibility and strength. The average 3HH content is preferably 7 mol% or less and more preferably 6 mol% or less. The average 3HH content is 0 mol% or more and may be 0.1 mol% or more or 1 mol% or more. The average 3HH content may be 0 mol%.

The weight percentage of the PHA fraction (II) in the PHA copolymer mixture is preferably from 45 to 99%, more preferably from 50 to 97%, even more preferably from 60 to 95%, and particularly preferably from 70 to 90%. The weight percentage of the PHA fraction (I) in the PHA copolymer mixture is preferably from 1 to 55%, more preferably from 3 to 50%, even more preferably from 5 to 40%, and particularly preferably from 10 to 30%. When the percentage of each fraction is in the above range, the resulting PHA copolymer mixture can be formed into a molded article having a good balance of flexibility and strength and, in addition, the PHA copolymer mixture can be granulated into particles with a desired particle size by spray drying performed at a relatively low drying temperature.

The average 3HH content in the total PHA copolymer mixture is preferably from 0.5 to 14 mol%. When the average 3HH content in the total PHA copolymer mixture is in this range, the PHA copolymer mixture can be formed into a molded article having a good balance of flexibility and strength. The average 3HH content is more preferably from 1 to 12 mol%, even more preferably from 1.5 to 10 mol%, still even more preferably from 2 to 8 mol%, and particularly preferably from 2.5 to 7 mol%.

### (MIBK Fractionation)

The PHA fraction (I) having a high average 3HH content can be separately collected from the PHA copolymer mixture by solvent fractionation which makes use of the difference in solubility in methyl isobutyl ketone (MIBK). PHAs having a higher 3HH content are more soluble in MIBK. Thus, the PHA fraction (I) can be obtained as a soluble fraction by dissolving all of the PHA copolymer mixture in high-temperature MIBK and then lowering the temperature of the solution to precipitate the PHA component having a lower 3HH content.

The following will describe the detailed procedures of the fractionation. First, about 100 mg of the PHA copolymer mixture is weighed into a screw-top test tube, to which 10 ml of MIBK is added and which is then closed with a cap. Subsequently, the test tube is heated at 140°C for about 0.5 to about 3 hours under shaking to dissolve the PHA copolymer mixture completely. After the complete dissolution, the test tube is left at room temperature for 1 minute to cool the solution to a temperature below the boiling point. All of the cooled solution is quickly transferred to a centrifuge tube, which is closed with a cap. The capped centrifuge tube is allowed to stand in a 35°C water bath for 30 minutes to precipitate a part of the dissolved matter. The precipitation is followed by centrifugation (at 9000 rpm for 5 minutes) to separate the precipitate, and all of the centrifuged supernatant is transferred to a container such as an aluminum dish. To the centrifuge tube containing the precipitate was added 10 ml of MIBK, and the contents of the centrifuge tube are mixed by means of a vortex mixer. The mixture is centrifuged (at 9000 rpm for 5 minutes), and the centrifuged supernatant is added to the same container such as an aluminum dish. At this point, a precipitate remains in the centrifuge tube. After that, the container is heated at 120°C for 30 minutes to evaporate MIBK and precipitate the dissolved matter in the centrifuged supernatant. The precipitate formed in the container and the precipitate remaining in the centrifuge tube are individually vacuum-dried at 100°C for 6 hours. The precipitate formed in the container is collected as the PHA fraction (I). The precipitate remaining in the centrifuge tube is collected as a "PHA composed mainly of the PHA fraction (II)".

### (Average 3HH Content Measurement Method Using Gas Chromatography)

The average 3HH content of the PHA copolymer mixture or the PHA fraction (I) can be measured as follows. To about 1 to about 20 mg of the dried PHA copolymer mixture or the dried PHA fraction (I) are added 1 ml of a sulfuric acid-methanol mixture (15:85) and 1 ml of chloroform, and the receptacle is hermetically closed. The contents of the receptacle are heated at 100°C for 140 minutes to obtain a methyl ester of a PHA decomposition product. After cooling, 0.5 ml of deionized water is added, and the contents of the receptacle are thoroughly mixed and then left until separation into aqueous and organic layers is completed. After that, the organic layer is separately collected, and the monomer unit composition of the PHA decomposition product in the organic layer is analyzed by capillary gas chromatography. The gas chromatograph used is GC-17A of Shimadzu Corporation, and the capillary column used is NEUTRA BOND-1 of GL Sciences Inc. (column length = 25 m, column inner diameter = 0.25 mm, liquid film thickness = 0.4 µm). The carrier gas used is He, the column inlet pressure is 100 kPa, and the volume of the injected sample is 1 µl. As for the temperature conditions, the temperature is 50°C at the beginning, increased to 200°C at a rate of 8°C/min, and then further increased from 200 to 290°C at a rate of 30°C/min. The average 3HH content of the PHA copolymer mixture or the PHA fraction (I) is calculated from peaks observed in the analysis under the above conditions.

### (Average 3HH Content Measurement Method Using DSC)

The average 3HH content of the PHA fraction (II) can be measured as follows. About 4 mg of the "PHA composed mainly of the PHA fraction (II)" is weighed out and subjected to measurement using a differential scanning calorimeter (DSC 8500 manufactured by PerkinElmer, Inc.). As for the temperature increase/decrease conditions, the temperature is held at 25°C for 1 minute, increased to 200°C, held at 200°C for 1 minute, decreased to 25°C, held at 25°C for 1 minute, increased to 100°C, held at 100°C for 60 minutes, decreased to 25°C, held at 25°C for 1 minute, and increased to 200°C. The rate of temperature increase/decrease is invariably 10°C/min. The temperature at which the highest melting peak is observed during the last temperature increase period is determined. The average 3HH content of the PHA fraction (II) is calculated based on the temperature at which the highest melting peak is observed and using a calibration curve created in advance from results of melting peak analysis of PHAs differing in 3HH content.

### (Method for Calculating Weight Percentage of Each Fraction in PHA Copolymer Mixture)

The weight percentage of each of the PHA fractions (I) and (II) in the PHA copolymer mixture can be determined as follows.

The calculated average 3HH contents of the PHA copolymer mixture, the PHA fraction (I), and the PHA fraction (II) are used to calculate in what weight percentages the PHA fractions (I) and (II) should be mixed to achieve the calculated average 3HH content of the PHA copolymer mixture, and thus the weight percentage of each of the PHA fractions (I) and (II) in the PHA copolymer mixture is determined.

### (PHA Copolymer Mixture-Producing Microorganism)

The microorganism used to produce the PHA copolymer mixture (this microorganism will be hereinafter also referred to as "PHA copolymer mixture-producing microorganism") is not limited to a particular type and may be any microorganism capable of fermentative production of the PHA copolymer mixture. The microorganism may be a wild strain that inherently accumulates PHAs, a mutant strain obtained by artificially mutating the wild strain, or a strain having a PHA accumulating ability attributed to an exogenous PHA synthase gene introduced by a genetic engineering technique.

Preferred examples of the PHA copolymer mixture-producing microorganism or the host of a transformed microorganism serving as the PHA copolymer mixture-producing microorganism include, but are not limited to, bacteria belonging to the genus *Ralstonia,* the genus *Cupriavidus,* the genus *Wautersia,* the genus *Aeromonas,* the genus *Escherichia,* the genus *Alcaligenes,* or the genus *Pseudomonas.* In terms of safety and PHA productivity, bacteria belonging to the genus *Ralstonia,* the genus *Cupriavidus,* the genus *Aeromonas,* or the genus *Wautersia* are more preferred. Even more preferred are bacteria belonging to the genus *Cupriavidus* or the genus *Aeromonas.* Still even more preferred are bacteria belonging to the genus *Cupriavidus.* Particularly preferred is *Cupriavidus necator.*

To efficiently produce two types of PHAs differing in average 3HH content, the PHA copolymer mixture-producing microorganism is preferably a microorganism having genes encoding two types of PHA synthases differing in polymerization activity for (R)-3-hydroxyhexanoyl-CoA. (R)-3-hydroxyhexanoyl-CoA is a precursor of 3HH structural units contained in PHAs. When the microorganism has genes encoding two types of PHA synthases differing in polymerization activity for (R)-3-hydroxyhexanoyl-CoA, a mixture of two types of PHAs differing in average 3HH content, i.e., the PHA copolymer mixture, can be produced in the cells of the microorganism by fermentation. The microorganism has at least two types of genes encoding PHA synthases differing in polymerization activity for (R)-3-hydroxyhexanoyl-CoA, and may have three or more types of such genes insofar as the microorganism is capable of fermentative production of the PHA copolymer mixture.

The two types of PHA synthases differing in polymerization activity for (R)-3-hydroxyhexanoyl-CoA are not limited to a particular combination. Preferably, the amino acid sequence identity between the two types of PHA synthases is 90% or less. The amino acid sequence identity is more preferably 80% or less and even more preferably 70% or less. PHA synthases are generally believed to form multimers such as dimers when performing their function. If the amino acid sequence identity between the two types of PHA synthases is more than 90%, the two types of PHA synthases could form hetero-dimers or the like and fail to produce the PHA copolymer mixture.

Of the two types of PHA synthases differing in polymerization activity for (R)-3-hydroxyhexanoyl-CoA, the PHA synthase having higher polymerization activity for (R)-3-hydroxyhexanoyl-CoA is a synthase that synthesizes a PHA copolymer having 3HB structural units and 3HH structural units. The gene encoding this PHA synthase will be referred to as the "gene (A)", while the gene encoding the other PHA synthase having lower polymerization activity for (R)-3-hydroxyhexanoyl-CoA than the PHA synthase encoded by the gene (A) will be referred to as the "gene (B)". In the PHA copolymer mixture-producing microorganism, the level of expression of the gene (A) is preferably lower than the level of expression of the gene (B). If the level of expression of the gene (A) is high, the weight percentage of the high-melting-point PHA which should be a main component ((PHA fraction (II)) could be below 45%.

The PHA copolymer mixture-producing microorganism is preferably a transformed microorganism in which the level of expression of the gene (A) is regulated to be lower than the level of expression of the gene (B). The regulation of the level of gene expression is not limited to using a particular method and may be accomplished, for example, by adjusting the site at which the target gene is introduced or by suitably selecting the type of the promoter of the target gene.

Examples of the promoter used to regulate the level of expression include, but are not limited to, a lac promoter, a lacUV5 promoter, a trp promoter, a trc promoter, and altered versions of these promoters. An exogenous promoter need not necessarily be used, and it is possible to use a promoter sequence inherently present on the genome DNA and in proximity to the site at which the target gene is inserted. Alternatively, the inherently present promoter sequence and an exogenous promoter may be used in combination.

Examples of the gene (A) include polyhydroxyalkanoate synthase genes derived from microorganisms of the genus *Aeromonas* and mutants of the polyhydroxyalkanoate synthase genes. A specific example is a gene encoding an amino acid sequence having a sequence identity of 90 to 100% with the amino acid sequence of any one of SEQ ID NOS: 1 to 8 (amino acid sequences of PHA synthase mutants derived from bacteria of the genus *Aeromonas).*

Other examples of the gene (A) include polyhydroxyalkanoate synthase genes identified by metagenome analysis and mutants of the polyhydroxyalkanoate synthase genes. A specific example is a gene encoding an amino acid sequence having a sequence identity of 90 to 100% with the amino acid sequence of SEQ ID NO: 9 or 44.

The sequence identity as mentioned above for the gene (A) is preferably 95% or more, more preferably 97% or more, particularly preferably 99% or more, and most preferably 99.5% or more.

Examples of the gene (B) include genes composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus.* A specific example is a gene encoding an amino acid sequence having a sequence identity of 90 to 100% with the amino acid sequence of SEQ ID NO: 10 or 11.

Other examples of the gene (B) include PHA synthase genes derived from bacteria of the genus *Chromobacterium* and mutants of the PHA synthase genes. A specific example is a gene encoding an amino acid sequence having a sequence identity of 90 to 100% with the amino acid sequence of SEQ ID NO: 12 or 13.

Still other examples of the gene (B) include polyhydroxyalkanoate synthase genes identified by metagenome analysis and mutants of the polyhydroxyalkanoate synthase genes. A specific example is a gene encoding an amino acid sequence having a sequence identity of 90 to 100% with the amino acid sequence of SEQ ID NO: 9 or 44.

The sequence identity as mentioned above for the gene (B) is preferably 95% or more, more preferably 97% or more, and particularly preferably 99% or more.

The combination of the genes (A) and (B) is selected such that the PHA synthase encoded by the gene (B) has lower polymerization activity for (R)-3-hydroxyhexanoyl-CoA than the PHA synthase encoded by the gene (A).

In order to efficiently produce a PHA copolymer mixture having a high average 3HH content, the PHA copolymer mixture-producing microorganism is preferably a microorganism having a gene encoding a protein that exhibits (R)-specific enoyl-CoA hydratase activity. (R)-specific enoyl-CoA hydratase has the function of converting hexenoyl-CoA to (R)-3-hydroxyhexanoyl-CoA in microbial cells. It is thus inferred that when the microorganism has a gene encoding a protein that exhibits (R)-specific enoyl-CoA hydratase activity, the presence of the gene increases the amount of conversion to (R)-3-hydroxyhexanoyl-CoA, leading to an increased average 3HH content in the PHA copolymer mixture produced.

The microorganism having a gene encoding a protein that exhibits (R)-specific enoyl-CoA hydratase activity may be a microorganism inherently having the gene or may be a microorganism into which the gene has been exogenously introduced by a genetic engineering technique.

When the microorganism inherently has a gene encoding a protein having (R)-specific enoyl-CoA hydratase activity and is, for example, *Cupriavidus necator,* examples of the gene include phaJ4a and phaJ4b genes.

Examples of the exogenous gene encoding a protein having (R)-specific enoyl-CoA hydratase activity include, but are not limited to: a gene derived from *Aeromonas caviae* and encoding (R)-specific enoyl-CoA hydratase having the amino acid sequence of SEQ ID NO: 14; a gene derived from *Cupriavidus necator* and encoding (R)-specific enoyl-CoA hydratase having the amino acid sequence of SEQ ID NO: 15 or 16; a multifunctional enzyme type 2 (MFE2) gene derived from *Yarrowia lipolytica* and encoding an enzyme having the amino acid sequence of SEQ ID NO: 17; an MFE2 gene derived from *Drosophila melanogaster* encoding an enzyme having the amino acid sequence of SEQ ID NO: 18; and a gene encoding a protein having a sequence identity of 85% or more, preferably 90% or more, more preferably 95% or more, particularly preferably 99% or more, with any one of the amino acid sequences of SEQ ID NOS: 14 to 18 and having (R)-specific enoyl-CoA hydratase activity.

In order to efficiently produce a PHA copolymer mixture having a high average 3HH content, the PHA copolymer mixture-producing microorganism may be a transformed microorganism that has been transformed to supply a larger amount of (R)-3-hydroxyhexanoyl-CoA to the intracellular PHA synthase than a wild strain of the microorganism.

An example of the transformed microorganism that has been transformed to increase the supply of (R)-3-hydroxyhexanoyl-CoA is a transformed microorganism that has been transformed to enhance the expression of a gene encoding a protein having (R)-specific enoyl-CoA hydratase activity. The enhancement of the expression of the gene can be accomplished by altering an expression regulatory sequence (a promoter sequence and/or an SD sequence) for the enhancement of the expression of the gene. This alteration is described, for example, in WO 2015/115619. Alternatively, as described in WO 2011/105379, the expression of the protein having (R)-specific enoyl-CoA hydratase activity can be enhanced by using a vector or integrating the gene into chromosomal DNA.

Another example of the transformed microorganism that has been transformed to increase the supply of (R)-3-hydroxyhexanoyl-CoA is a transformed microorganism that has been transformed to more strongly inhibit degradation of an intermediate metabolite having six carbon atoms in β-oxidation of an oil or a fatty acid than a wild strain of the microorganism. It is inferred that the inhibition of degradation of an intermediate metabolite having six carbon atoms in β-oxidation results in an increase in the supply of (R)-3-hydroxyhexanoyl-CoA and hence an increased average 3HH content in the PHA copolymer mixture produced.

An example of the transformed microorganism that has been transformed to inhibit degradation of an intermediate metabolite having six carbon atoms in β-oxidation of an oil or a fatty acid is a transformed microorganism that has been transformed to inhibit the expression of a gene encoding a β-ketothiolase enzyme having thiolysis activity for β-ketohexanoyl-CoA which is β-ketoacyl-CoA having six carbon atoms. Such a transformed microorganism is described, for example, in Patent Literature 5. This transformed microorganism may be one that has undergone both the transformation for inhibiting the expression of a gene encoding a β-ketothiolase enzyme having thiolysis activity for β-ketohexanoyl-CoA and the previously described transformation for enhancing the expression of a gene encoding a protein having (R)-specific enoyl-CoA hydratase activity.

Examples of the gene encoding a β-ketothiolase enzyme include, but are not limited to, bktB and A1528 genes, and a specific example is, but not limited to, a gene encoding a β-ketothiolase enzyme having an amino acid sequence having a sequence identity of 90 to 100% with the amino acid sequence of SEQ ID NO: 19 or 20. The sequence identity is preferably 95% or more, more preferably 97% or more, and particularly preferably 99% or more.

Examples of methods for inhibiting the expression of the β-ketothiolase enzyme-encoding gene include: a method in which the enzyme gene is completely deleted from the transformed microorganism; a method in which a quite different gene such as a drug-resistant gene is inserted into the sequence of the enzyme gene; and a method in which a part of the sequence of the enzyme gene (the part is preferably a domain responsible for the enzyme activity) is mutated by deletion, substitution, addition, or insertion. Examples of the gene disruption process include a homologous recombination technique using a vector containing a gene or DNA for disruption and a technique using a transposon. Examples of other disruption methods include known techniques such as genome editing using a CRISPR/Cas (e.g., Cas9) system or TALEN for disrupting the target gene (Y. Wang et al., ACS Synth Biol. 2016, 5(7): 721-732; Bogdanove and Voytas, Science, 333: 1843-1846, 2011; Jinek et al., Science, 337: 816-821, 2012; Shalem et al., Science, 343: 84-87, 2014; and Wang et al., Science, 343: 80-84, 2014). For example, in the case of the CRISPR/Cas9 system, the guide RNA (gRNA) has a sequence capable of binding to a part of the base sequence of the β-ketothiolase gene to be disrupted and serves to carry the Cas9 to the target. Alternatively, a base sequence neighboring the target gene may be mutated by deletion, substitution, addition, or insertion to reduce the transcription-translation efficiency of the gene or the stability of the mRNA and thereby eliminate or reduce the enzyme activity.

In the case where the PHA copolymer mixture-producing microorganism has an introduced exogenous gene, the introduced gene may be present on a chromosome possessed by the microorganism used as the host or on DNA such as a plasmid or megaplasmid possessed by the microorganism used as the host. In view of retention of the introduced gene, the introduced gene is preferably present on a chromosome or megaplasmid possessed by the microorganism and more preferably present on a chromosome possessed by the microorganism. In the case of enhancing the level of expression of a gene inherently possessed by the microorganism used as the host, a base sequence upstream of the gene may be mutated, for example, by substitution, deletion, or addition to enhance the level of expression of the gene.

Methods for modifying DNA of a microorganism by site-specific substitution with or insertion of given DNA or deleting a given site of the DNA of the microorganism are known to those skilled in the art, and any of the known methods can be used to produce the transformed microorganism of the present invention. Typical examples of the methods include, but are not limited to: a method using a transposon and the mechanism of homologous recombination (Ohman et al., J. Bacteriol., vol. 162: p. 1068 (1985)); a method based on site-specific integration induced by the mechanism of homologous recombination and on loss due to second homologous recombination (Noti et al., Methods Enzymol., vol. 154, p. 197 (1987)); and a method in which a sacB gene derived from *Bacillus subtilis* is allowed to coexist and thus in which a microbial strain having lost a gene due to second homologous recombination is easily isolated as a sucrose-supplemented medium-resistant strain (Schweizer, Mol. Microbiol., vol. 6, p. 1195 (1992); Lenz et al., J. Bacteriol., vol. 176, p. 4385 (1994)). The method for introducing a vector into cells is not limited to a particular technique, and examples of the method include calcium chloride transformation, electroporation, polyethylene glycol transformation, and spheroplast transformation.

For gene cloning or gene recombination, a technique as described in Sambrook, J. et al., Molecular Cloning: A Laboratory Manual, Cold Spring Harbor Laboratory Press (1989 or 2001) can be used.

The promoter for expressing the introduced gene is not limited to a particular type. Examples of promoters that can be used include: a phaC1 gene promoter and a phaP1 gene promoter of *Cupriavidus necator;* a trp promoter, a lac promoter, a lacUV5 promoter, a trc promoter, a tic promoter, and a tac promoter which are derived from *Escherichia coli;* an artificially prepared lacN17 promoter having an *Escherichia coli*-derived altered base sequence of SEQ ID NO: 21; and an artificially prepared lacN19 promoter having an *Escherichia coli-*derived altered base sequence of SEQ ID NO: 22.

### (Culture of Microorganism)

Culturing the PHA copolymer mixture-producing microorganism allows the microbial cells to accumulate the PHA copolymer mixture. The culture of the PHA copolymer mixture-producing microorganism can be conducted by an ordinary microbial culture method, and it is sufficient that the PHA copolymer mixture-producing microorganism be cultured in a culture medium containing a suitable carbon source. There are no particular limitations on the composition of the culture medium, the way of adding the carbon source, the scale of the culture, the conditions of aeration and stirring, the culture temperature, the culture time, etc. It is preferable to add the carbon source to the culture medium continuously or intermittently.

The carbon source used for the culture may be any carbon source that can be assimilated by the PHA copolymer mixture-producing microorganism. Examples of the carbon source include, but are not limited to: sugars such as glucose, fructose, sucrose, and xylose; oils such as palm and palm kernel oils (including palm olein, palm double olein, and palm kernel olein which are low-melting-point fractions obtained through fractionation of palm oil and palm kernel oil), corn oil, coconut oil, olive oil, soybean oil, rapeseed oil, and Jatropha oil; fractions of these oils; by-products formed during refining of these oils; fatty acids such as lauric acid, oleic acid, stearic acid, palmitic acid, and myristic acid; derivatives of these fatty acids; and glycerol. In the case where the PHA copolymer mixture-producing microorganism can assimilate gases such as carbon dioxide, carbon monoxide, and methane or alcohols such as methanol and ethanol, any of these gases or alcohols can be used as the carbon source. Preferably, the carbon source contains an oil (in particular, a vegetable oil) or a fatty acid.

In the production of the PHA copolymer mixture, it is preferable to culture the microorganism using a culture medium containing the carbon source as described above and other nutrient sources including a nitrogen source, an inorganic salt, and another organic nutrient source. Examples of the nitrogen source include, but are not limited to: ammonia; ammonium salts such as ammonium chloride, ammonium sulfate, and ammonium phosphate; peptone; meat extracts; and yeast extracts. Examples of the inorganic salt include potassium dihydrogen phosphate, sodium dihydrogen phosphate, magnesium phosphate, magnesium sulfate, and sodium chloride. Examples of the other organic nutrient source include: amino acids such as glycine, alanine, serine, threonine, and proline; and vitamins such as vitamin B1, vitamin B12, and vitamin C.

After the PHA copolymer mixture-producing microorganism is cultured for an adequate time to allow the microbial cells to accumulate the PHA copolymer mixture, the PHA copolymer mixture is collected from the microbial cells using a known method. The method for collection is not limited to a particular technique. From the industrial point of view, it is preferable to collect the PHA copolymer mixture through separation and purification using an aqueous system which has a low environmental impact. For example, the cultured cells may be disrupted by application of a mechanical shear force or by the use of a surfactant, an alkali, or an enzyme to obtain a disrupted cell solution in which cellular components other than the PHAs are dissolved in water. The PHA copolymer mixture can be collected by separating the PHA copolymer mixture from the aqueous phase through filtration or centrifugation of the disrupted cell solution and then drying the separated PHA copolymer mixture.

### (Spray Drying)

According to a preferred aspect of the present invention, microbial culture is followed by disrupting the microbial cells, purifying a PHA copolymer mixture, and obtaining an aqueous suspension of the PHA copolymer mixture, and then the aqueous suspension is subjected to spray drying, by which a powder of the PHA copolymer mixture can be obtained. Since PHAs can be obtained as a powder, a PHA material with good handleability can be obtained efficiently.

The "aqueous suspension of a PHA copolymer mixture" refers to a liquid containing an aqueous medium in which is dispersed the PHA copolymer mixture from which cell-derived components other than the PHAs have been removed and which is of high purity. The aqueous suspension contains water as the aqueous medium and may further contain a water-miscible organic solvent (such as ethanol, ethanol, or acetone).

The concentration of the PHA copolymer mixture in the aqueous suspension can be set in view of productivity improvement in the spray drying and the fluidity of the aqueous suspension. To be specific, the concentration of the PHA copolymer mixture is preferably from 30 to 65 wt% and more preferably from 40 to 60 wt%.

The aqueous suspension may further contain a dispersant. Specific examples of the dispersant include alkylene oxide dispersants as mentioned in WO 2021/251049 and polyvinyl alcohol.

The aqueous suspension can be obtained, for example, as follows. The PHA copolymer mixture-producing microorganism is cultured to allow the microbial cells to accumulate the PHA copolymer mixture, and then the cells are subjected to a disruption process to obtain a disrupted cell solution. Subsequently, the disrupted cell solution is subjected, if necessary, to a process such as filtration or centrifugation for water removal, followed by a purification process to decompose or remove cell-derived components other than the PHAs. The resulting PHA copolymer mixture is washed with a liquid such as water if necessary, and then the water-containing aqueous medium is added or removed if necessary to adjust the concentration of the PHA copolymer mixture. In this way, the aqueous suspension of the PHA copolymer mixture can be obtained. One example of references for details of the procedures is WO 2010/067543.

The aqueous suspension of the PHA copolymer mixture can be spray-dried to obtain a powder of the PHA copolymer mixture. The spray drying can be accomplished, for example, by a method in which fine droplets of the aqueous suspension of the PHA copolymer mixture are fed into a dryer and dried in contact with hot air in the dryer.

The temperature of the hot air used in the spray drying is not limited to a particular value and can be chosen as appropriate in the range of 100 to 300°C. In terms of reducing energy consumption during the spray drying, the temperature of the hot air is preferably up to 200°C and more preferably up to 180°C. The temperature of the air discharged from the spray dryer (discharged air temperature) is not limited to a particular value either. In terms of controlling the particle size of the powder by fusing the surfaces of the PHA particles to one another, the discharged air temperature is preferably 80°C or higher and more preferably 90°C or higher.

According to a preferred aspect, a PHA powder having a large particle size can be obtained by spray drying performed at a lowered drying temperature. This can result in a reduction in the cost (including equipment cost and utility cost) required for the drying step.

In the following items, preferred aspects of the present disclosure are listed. The present invention is not limited to the following items.

### [Item 1]

A method for producing a polyhydroxyalkanoate copolymer mixture, the method including the step of culturing a microorganism that produces the polyhydroxyalkanoate copolymer mixture, wherein
the polyhydroxyalkanoate copolymer mixture contains:
   a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
   a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.

### [Item 2]

The method according to item 1, wherein an average 3-hydroxyhexanoate content in the total polyhydroxyalkanoate copolymer mixture is from 0.5 to 14 mol%.

### [Item 3]

The method according to item 1 or 2, wherein the microorganism has genes encoding two types of polyhydroxyalkanoate synthases differing in polymerization activity for (R)-3-hydroxyhexanoyl-CoA.

### [Item 4]

The method according to item 3, wherein an amino acid sequence identity between the two types of polyhydroxyalkanoate synthases differing in polymerization activity for (R)-3-hydroxyhexanoyl-CoA is 90% or less.

### [Item 5]

The method according to item 3 or 4, wherein the genes encoding the two types of polyhydroxyalkanoate synthases differing in polymerization activity for (R)-3-hydroxyhexanoyl-CoA include:
a gene (A) encoding a polyhydroxyalkanoate synthase that synthesizes a polyhydroxyalkanoate copolymer having 3-hydroxybutyrate structural units and 3-hydroxyhexanoate structural units; and
a gene (B) encoding a polyhydroxyalkanoate synthase having lower polymerization activity for (R)-3-hydroxyhexanoyl-CoA than the polyhydroxyalkanoate synthase encoded by the gene (A).

### [Item 6]

The method according to item 5, wherein in the microorganism, a level of expression of the gene (A) is regulated to be lower than a level of expression of the gene (B).

### [Item 7]

The method according to item 5 or 6, wherein the gene (A) is a polyhydroxyalkanoate synthase gene derived from a microorganism of the genus *Aeromonas* or a mutant of the polyhydroxyalkanoate synthase gene.

### [Item 8]

The method according to item 7, wherein the gene (A) is a gene encoding an amino acid sequence having a sequence identity of 99.5 to 100% with an amino acid sequence of any one of SEQ ID NOS: 1 to 8.

### [Item 9]

The method according to any one of items 5 to 8, wherein the gene (B) is composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus.*

### [Item 10]

The method according to item 9, wherein the gene (B) is a gene encoding an amino acid sequence having a sequence identity of 90 to 100% with an amino acid sequence of SEQ ID NO: 10 or 11.

### [Item 11]

The method according to any one of items 5 to 8, wherein the gene (B) is a polyhydroxyalkanoate synthase gene derived from a microorganism of the genus *Chromobacterium* or a mutant of the polyhydroxyalkanoate synthase gene.

### [Item 12]

The method according to item 11, wherein the gene (B) is a gene encoding an amino acid sequence having a sequence identity of 90 to 100% with an amino acid sequence of SEQ ID NO: 12 or 13.

### [Item 13]

The method according to any one of items 5 to 12, wherein either one of the genes (A) and (B) is a gene encoding an amino acid sequence having a sequence identity of 90 to 100% with an amino acid sequence of SEQ ID NO: 9 or 44.

### [Item 14]

The method according to any one of items 1 to 13, wherein the microorganism is a microorganism having a gene encoding a protein that exhibits (R)-specific enoyl-CoA hydratase activity.

### [Item 15]

The method according to any one of items 1 to 14, wherein the microorganism is a transformed microorganism that has been transformed to supply a larger amount of (R)-3-hydroxyhexanoyl-CoA to an intracellular polyhydroxyalkanoate synthase than a wild strain of the microorganism.

### [Item 16]

The method according to item 15, wherein the transformed microorganism is a microorganism that has been transformed to enhance expression of a gene encoding a protein that exhibits (R)-specific enoyl-CoA hydratase activity.

### [Item 17]

The method according to item 15 or 16, wherein the transformed microorganism is a microorganism that has been transformed to inhibit expression of a gene encoding a β-ketothiolase enzyme having thiolysis activity for β-ketohexanoyl-CoA which is β-ketoacyl-CoA having six carbon atoms.

### [Item 18]

The method according to any one of items 1 to 17, wherein a carbon source containing an oil or a fatty acid is added in the culturing step.

### [Item 19]

The method according to any one of items 1 to 18, wherein the microorganism belongs to the genus *Cupriavidus* or is a transformant of a microorganism of the genus *Cupriavidus.*

### [Item 20]

The method according to item 19, wherein the microorganism is *Cupriavidus necator* or a transformant of *Cupriavidus necator.*

### [Item 21]

The method according to any one of items 1 to 20, further including the steps of:
after culturing the microorganism, disrupting cells of the microorganism, purifying the polyhydroxyalkanoate copolymer mixture, and obtaining an aqueous suspension of the polyhydroxyalkanoate copolymer mixture; and
spray-drying the aqueous suspension to obtain a powder of the polyhydroxyalkanoate copolymer mixture.

### [Item 22]

A transformed microorganism that produces a polyhydroxyalkanoate copolymer mixture, the transformed microorganism having genes encoding two types of polyhydroxyalkanoate synthases differing in polymerization activity for (R)-3-hydroxyhexanoyl-CoA, wherein
the polyhydroxyalkanoate copolymer mixture contains:
   a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
   a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.

### Examples

Hereinafter, the present invention will be described in more detail using examples. The present invention is not limited to the examples. The overall genetic manipulation can be carried out, for example, in a manner as taught in Molecular Cloning (Cold Spring Harbor Laboratory Press (1989)). The enzymes, cloning hosts, and other materials used in the genetic manipulation can be purchased from market suppliers and used according to the instructions given by the suppliers. The enzymes are not limited to particular types and may be any enzymes that can be used for genetic manipulation.

### (Microbial Strain Preparation Example 1)

Preparation of PHA copolymer mixture-producing microbial strain (1)

First, a plasmid for PHA synthase gene disruption was prepared. The preparation was done as follows.

PCR using synthetic oligo DNA was carried out to obtain a DNA fragment (SEQ ID NO: 23) having base sequences upstream and downstream of the phaC1 structural gene (PHA synthase gene) of *Cupriavidus necator* H16. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+phaC1UD for PHA synthase gene disruption was prepared.

Subsequently, a PHA synthase gene-disrupted strain was prepared using the plasmid vector pNS2X-sacB+phaC1UD for PHA synthase gene disruption. The preparation was done as follows.

*Escherichia coli* S17-1 (ATCC 47055) was transformed with the plasmid vector pNS2X-sacB+phaC1UD for PHA synthase gene disruption, and the resulting transformed microorganism was co-cultured with KNK005 trc-phaJ4b/ΔphaZ1,2,6 (hereinafter also referred to as "KNK005dZ/trc-J4b") on Nutrient Agar (manufactured by Difco Laboratories) to effect conjugal transfer.

The KNK005dZ/trc-J4b is a strain in which: the phaZ1, phaZ2, and phaZ6 genes on the chromosome of *Cupriavidus necator* H16 are deleted; the PHA synthase gene on the chromosome is substituted with an altered PHA synthase gene derived from the genus *Aeromonas* (a gene encoding a PHA synthase having the amino acid sequence of SEQ ID NO: 2, i.e., N149S/D171G mutant (NSDG) gene); and the expression of the (R)-specific enoyl-CoA hydratase gene (phaJ4b gene) on the chromosome is enhanced. This strain can be prepared according to the method described in WO 2015/115619.

The culture fluid obtained as above was inoculated into a Simmons agar medium (2 g/L sodium citrate, 5 g/L sodium chloride, 0.2 g/L magnesium sulfate heptahydrate, 1 g/L ammonium dihydrogen phosphate, 1 g/L potassium dihydrogen phosphate, 15 g/L agar, pH = 6.8) containing 250 mg/L kanamycin, and strains grown on the agar medium were selectively collected. Thus, a strain having the plasmid integrated into the chromosome of the KNK005dZ/trc-J4b was obtained. This strain was cultured in Nutrient Broth (manufactured by Difco Laboratories) for two generations, after which the culture broth was diluted and applied onto Nutrient Agar containing 15% sucrose. Strains grown on the Nutrient Agar were obtained as strains from which the plasmid was lost. PCR and analysis using a DNA sequencer were further carried out to isolate one strain in which the PHA synthase gene on the chromosome was deleted. This gene-disrupted strain was named "KNK005dZ/dNSDG/trc-J4b".

A plasmid for PHA synthase gene introduction was also prepared. The preparation was done as follows.

PCR using synthetic oligo DNA was carried out to obtain a DNA fragment (SEQ ID NO: 24) having base sequences upstream and downstream of the B1168 structural gene (whose function is unknown) of *Cupriavidus necator* H16, having a trp promoter, and having a base sequence of a gene encoding a PHA synthase having the amino acid sequence of SEQ ID NO: 12. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1168U-trp-phaCcsA479W-B1168D for PHA synthase gene introduction was prepared.

Next, the plasmid vector pNS2X-sacB+B1168U-trp-phaCcsA479W-B1168D for PHA synthase gene introduction was introduced into the KNK005dZ/dNSDG/trc-J4b by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 12 were introduced at a site where the B1168 gene was originally present. The strain thus obtained was named "KNK005dZ/dNSDG/trc-J4b/B1168::trp-phaCesA479W". It has been confirmed that the deletion of the B1168 gene does not affect the growth or PHA biosynthesis of the strain.

Another plasmid for PHA synthase gene introduction was also prepared. The preparation was done as follows.

PCR using synthetic oligo DNA was carried out to obtain a DNA fragment (SEQ ID NO: 25) having a base sequence upstream of the A2712 structural gene (whose function is unknown) of *Cupriavidus necator* H16, having a part of the base sequence of the A2712 structural gene, and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+A2712U-phaCamSGLVNE-A2712 for PHA synthase gene introduction was prepared.

Next, a PHA synthase gene-introduced strain was prepared using the plasmid vector pNS2X-sacB+A2712U-phaCamSGLVNE-A2712 for PHA synthase gene introduction. The preparation was done as follows.

The plasmid vector pNS2X-sacB+A2712U-phaCamSGLVNE-A2712 for PHA synthase gene introduction was introduced into the KNK005dZ/dNSDG/trc-J4b/B1168::trp-phaCcsA479W by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 2 was introduced upstream of the A2712 gene on the chromosome. The strain thus obtained was named "KNK005dZ/dNSDG/trc-J4b/B1168::trp-phaCcsA479W/PA2712-phaCamSGLVNE" (hereinafter also referred to as "PHA copolymer mixture-producing microbial strain (1)").

The PHA copolymer mixture-producing microbial strain (1) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene (phaJ4b gene) on the chromosome is enhanced; and a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced.

### (Microbial Strain Preparation Example 2)

Preparation of PHA copolymer mixture-producing microbial strain (2)

First, a plasmid for bktB gene disruption was prepared. The preparation was done as follows.

PCR using synthetic oligo DNA was carried out to obtain a DNA fragment (SEQ ID NO: 26) having base sequences upstream and downstream of the bktB structural gene (β-ketothiolase enzyme gene) of *Cupriavidus necator* H16. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBUD for bktB gene disruption was prepared.

Next, a bktB gene-disrupted strain was prepared using the plasmid vector pNS2X-sacB+bktBUD for bktB gene disruption. The preparation was done as follows.

The plasmid vector pNS2X-sacB+bktBUD for bktB gene disruption was introduced into the KNK005dZ/dNSDG/trc-J4b/B1168::trp-phaCcsA479W/PA2712-phaCamSGLVNE (PHA copolymer mixture-producing microbial strain (1)) by procedures using conjugal transfer as described above. The subsequent culture and selection on Nutrient Agar containing 15% sucrose were carried out as described above to isolate one strain in which the bktB gene on the chromosome was deleted. The strain thus obtained was named "KNK005dZ/dNSDG/trc-J4b/B1168::trp-phaCesA479W/PA2712-phaCamSGLVNE/dbktB" (hereinafter also referred to as "PHA copolymer mixture-producing microbial strain (2)").

The PHA copolymer mixture-producing microbial strain (2) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced; and the bktB gene on the chromosome is deleted.

### (Microbial Strain Preparation Example 3)

Preparation of PHA copolymer mixture-producing microbial strain (3)

First, a plasmid for PHA synthase gene introduction was prepared. The preparation was done as follows.

PCR using synthetic oligo DNA was carried out to obtain a DNA fragment (SEQ ID NO: 27) having base sequences upstream and downstream of the bktB structural gene (β-ketothiolase enzyme gene) of *Cupriavidus necator* H16 and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-phaCamSGLVNE-bktBD for PHA synthase gene was prepared.

Next, a PHA synthase gene-introduced strain was prepared using the plasmid vector pNS2X-sacB+bktBU-phaCamSGLVNE-bktBD for PHA synthase gene introduction. The preparation was done as follows.

The plasmid vector pNS2X-sacB+bktBU-phaCamSGLVNE-bktBD for PHA synthase gene introduction was introduced into the KNK005dZ/dNSDG/trc-J4b/B1168::trp-phaCcsA479W by procedures using conjugal transfer as described above. The subsequent culture and selection on Nutrient Agar containing 15% sucrose were carried out as described above to isolate one strain in which the bktB gene on the chromosome was deleted and in which the gene encoding a PHA synthase having the amino acid sequence of SEQ ID NO: 2 was introduced at a site where the bktB gene was originally present. The strain thus obtained was named "KNK005dZ/dNSDG/trc-J4b/B1168::trp-phaCcsA479W/bktB::phaCamSGLVNE" (hereinafter also referred to as "PHA copolymer mixture-producing microbial strain (3)").

The PHA copolymer mixture-producing microbial strain (3) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced; and the bktB gene on the chromosome is deleted.

### (Microbial Strain Preparation Example 4)

Preparation of PHA copolymer mixture-producing microbial strain (4)

First, a plasmid for PHA synthase gene introduction was prepared. The preparation was done as follows.

PCR using synthetic oligo DNA was carried out to obtain a DNA fragment (SEQ ID NO: 28) having a base sequence upstream of the A2712 structural gene (whose function is unknown) of *Cupriavidus necator* H16, having a part of the base sequence of the A2712 structural gene, having a lac promoter, and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+A2712U-lac-phaCamSGLVNE-A2712 for PHA synthase gene introduction was prepared.

Next, a PHA synthase gene-introduced strain was prepared using the plasmid vector pNS2X-sacB+A2712U-lac-phaCamSGLVNE-A2712 for PHA synthase gene introduction. The preparation was done as follows.

The plasmid vector pNS2X-sacB+A2712U-lac-phaCamSGLVNE-A2712 for PHA synthase gene introduction was introduced into the KNK005dZ/dNSDG/trc-J4b/B1168::trp-phaCcsA479W by procedures using conjugal transfer as described above. The subsequent culture and selection on Nutrient Agar containing 15% sucrose were carried out as described above to isolate one strain in which the lac promoter and the gene encoding a PHA synthase having the amino acid sequence of SEQ ID NO: 2 were introduced upstream of the A2712 gene on the chromosome. The strain thus obtained was named "KNK005dZ/dNSDG/trc-J4b/B1168::trp-phaCcsA479W/PA2712-lac-phaCamSGLVNE" (hereinafter also referred to as "PHA copolymer mixture-producing microbial strain (4)").

The PHA copolymer mixture-producing microbial strain (4) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; and a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced.

### (Microbial Strain Preparation Example 5)

Preparation of PHA copolymer mixture-producing microbial strain (5)

First, a PHA synthase gene-disrupted strain was prepared using the plasmid vector pNS2X-sacB+phaC1UD for PHA synthase gene disruption. The preparation was done as follows.

The plasmid vector pNS2X-sacB+phaC1UD for PHA synthase gene disruption was introduced into KNK005dZ/trc-J4b/dbktB by procedures using conjugal transfer as described above.

The KNK005dZ/trc-J4b/dbktB is a strain in which: the phaZ1, phaZ2, and phaZ6 genes on the chromosome of *Cupriavidus necator* H16 are deleted; the PHA synthase gene on the chromosome is substituted with an altered PHA synthase gene derived from the genus *Aeromonas* (a gene encoding a PHA synthase having the amino acid sequence of SEQ ID NO: 2, i.e., N149S/D171G mutant ((NSDG) gene); the expression of the (R)-specific enoyl-CoA hydratase gene on the chromosome is enhanced; and the bktB structural gene is deleted. This strain can be prepared according to the method described in WO 2019/142845.

The subsequent culture and selection on Nutrient Agar containing 15% sucrose were carried out as described above to isolate one strain in which the PHA synthase gene on the chromosome was deleted. This gene-disrupted strain was named "KNK005dZ/dNSDG/trc-J4b/dbktB".

A plasmid for PHA synthase gene introduction was also prepared. The preparation was done as follows.

PCR using synthetic oligo DNA was carried out to obtain a DNA fragment (SEQ ID NO: 29) having a base sequence upstream of the B1185 structural gene (whose function is unknown) of *Cupriavidus necator* H16, having a part of the base sequence of the B1185 structural gene, having a trp promoter, and having a base sequence of a gene encoding a PHA synthase having the amino acid sequence of SEQ ID NO: 12. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1185U-trp-phaCcsA479W-B1185 for PHA synthase gene introduction was prepared.

Next, a PHA synthase gene-introduced strain was prepared using the plasmid vector pNS2X-sacB+B1185U-trp-phaCcsA479W-B1185 for PHA synthase gene introduction. The preparation was done as follows.

The plasmid vector pNS2X-sacB+B1185U-trp-phaCcsA479W-B1185 for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the trp promoter and the gene encoding a PHA synthase having the amino acid sequence of SEQ ID NO: 12 were introduced upstream of the B1185 gene on the chromosome. The strain thus obtained was named "KNK005dZ/dNSDG/trc-J4b/dbktB/PB1185-trp-phaCcsA479W".

Furthermore, a PHA synthase gene-introduced strain was prepared using the plasmid vector pNS2X-sacB+A2712U-phaCamSGLVNE-A2712 for PHA synthase gene introduction. The preparation was done as follows.

The plasmid vector pNS2X-sacB+A2712U-phaCamSGLVNE-A2712 for PHA synthase gene introduction was introduced into the KNK005dZ/dNSDG/trc-J4b/dbktB/PB1185-trp-phaCcsA479W by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 2 was introduced upstream of the A2712 gene on the chromosome. The strain thus obtained was named "KNK005dZ/dNSDG/trc-J4b/dbktB/PB1185-trp-phaCcsA479W/PA2712-phaCamSGLVNE" (hereinafter also referred to as "PHA copolymer mixture-producing microbial strain (5)").

The PHA copolymer mixture-producing microbial strain (5) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced.

### (Microbial Strain Preparation Example)

Preparation of PHA copolymer mixture-producing microbial strain (6)

First, a plasmid for PHA synthase gene introduction was prepared. The preparation was done as follows.

PCR using synthetic oligo DNA was carried out to obtain a DNA fragment (SEQ ID NO: 30) having a base sequence upstream of the B1185 structural gene (whose function is unknown) of *Cupriavidus necator* H16, having a part of the base sequence of the B1185 structural gene, and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1185U-phaCamSGLVNE-B1185 for PHA synthase gene introduction was prepared.

Next, a PHA synthase gene-introduced strain was prepared using the plasmid vector pNS2X-sacB+B1185U-phaCamSGLVNE-B1185 for PHA synthase gene introduction. The preparation was done as follows.

The plasmid vector pNS2X-sacB+B1185U-phaCamSGLVNE-B1185 for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 2 was introduced upstream of the B1185 gene on the chromosome. The strain thus obtained was named "KNK005dZ/dNSDG/trc-J4b/dbktB/PB1185-phaCamSGLVNE".

A plasmid for PHA synthase gene expression was also prepared. The preparation was done as follows. PCR using synthetic oligo DNA was carried out to obtain a DNA fragment (SEQ ID NO: 31) having a lacUV5 promoter which is an altered lac promoter of *Escherichia coli.* The DNA fragment was digested with restriction enzymes EcoRI and MunI, and the resulting DNA fragment was joined to a plasmid vector pCUP2 which is described in WO 2007/049716 and which was cleaved with MunI. A conjugate was selected in which the DNA fragment was joined to the pCUP2 in such an orientation that the restriction enzyme SpeI-recognition sequence of the pCUP2 was located downstream of the lacUV5 promoter. In this manner, pCUP2-lacUV5 was obtained. Next, PCR using synthetic oligo DNA was carried out to obtain a DNA fragment (SEQ ID NO: 32) having a base sequence of a gene encoding a PHA synthase having the amino acid sequence of SEQ ID NO: 12. The DNA fragment was digested with restriction enzymes EcoRI and SpeI, and the resulting DNA fragment was joined to the pCUP2-lacUV5 cleaved with MunI and SpeI. In this manner, a plasmid pCUP2-lacUV5-phaCcsA479W for PHA synthase gene expression was obtained.

Next, the plasmid pCUP2-lacUV5-phaCcsA479W for PHA synthase gene expression was introduced into the KNK005dZ/dNSDG/trc-J4b/dbktB/PB1185-phaCamSGLVNE. The resulting strain was named "KNK005dZ/dNSDG/trc-J4b/dbktB/PB1185-phaCamSGLVNE/pCUP2-lacUV5-phaCcsA479W" (hereinafter also referred to as "PHA copolymer mixture-producing microbial strain (6)").

The introduction of the plasmid vector into the cells was carried out by electroporation as described below. The gene introduction device used was Gene Pulser manufactured by Bio-Rad Laboratories, Inc., and the cuvette used was a 0.2-cm-gap cuvette also manufactured by Bio-Rad Laboratories, Inc. The cuvette was charged with 400 µl of competent cells and 20 µl of the expression vector and set on the pulse device, by which electric pulse was applied to the contents of the cuvette at a capacitance of 25 µF, a voltage of 1.5 kV, and a resistance value of 800 Ω. After the pulse application, the cell-containing fluid in the cuvette was subjected to shake culture in Nutrient Broth (manufactured by Difco Laboratories) at 30°C for 3 hours and then to culture on a selection plate (Nutrient Agar manufactured by Difco Laboratories, containing 100 mg/L kanamycin) at 30°C for 2 days. The PHA copolymer mixture-producing microbial strain (6) grown through these procedures was collected.

The PHA copolymer mixture-producing microbial strain (6) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from the genus *Chromobacterium* and having the amino acid sequence of SEQ ID NO: 12 are introduced.

### (Microbial Strain Preparation Example)

Preparation of PHA copolymer mixture-producing microbial strain (7)

First, a plasmid for PHA synthase gene introduction was prepared. The preparation was done as follows.

PCR using synthetic oligo DNA was carried out to obtain a DNA fragment (SEQ ID NO: 33) having base sequences upstream and downstream of the bktB structural gene (β-ketothiolase enzyme gene) of *Cupriavidus necator* H16, having a lacN19 promoter, and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-lacN19-phaCamSGLVNE-bktBD for PHA synthase gene introduction was prepared.

Next, a PHA synthase gene-introduced strain was prepared using the plasmid vector pNS2X-sacB+bktBU-lacN19-phaCamSGLVNE-bktBD for PHA synthase gene introduction. The preparation was done as follows.

The plasmid vector pNS2X-sacB+bktBU-lacN19-phaCamSGLVNE-bktBD for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the bktB gene on the chromosome was deleted and in which the lacN19 promoter and the gene encoding a PHA synthase having the amino acid sequence of SEQ ID NO: 2 were introduced at a site where the bktB gene was originally present. The strain thus obtained was named "KNK005dZ/dNSDG/trc-J4b/bktB::lacN19-phaCamSGLVNE".

Another plasmid for PHA synthase gene introduction was also prepared. The preparation was done as follows.

PCR using synthetic oligo DNA was carried out to obtain a DNA fragment (SEQ ID NO: 34) having base sequences upstream and downstream of the B1168 structural gene (whose function is unknown) of *Cupriavidus necator* H16 and having a base sequence of a gene encoding a PHA synthase having the amino acid sequence of SEQ ID NO: 11. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1168U-AmNSRe12A506M-B1168D for PHA synthase gene introduction was prepared.

Next, the plasmid vector pNS2X-sacB+B1168U-AmNSRe12A506M-B1168D for PHA synthase gene introduction was introduced into the KNK005dZ/dNSDG/trc-J4b/bktB::lacN19-phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the gene encoding a PHA synthase having the amino acid sequence of SEQ ID NO: 11 was introduced at a site where the B1168 gene was originally present. The strain thus obtained was named "KNK005dZ/dNSDG/trc-J4b/bktB::lacN19-phaCamSGLVNE/B1168::AmNSRe12A506M" (hereinafter also referred to as "PHA copolymer mixture-producing microbial strain (7)").

The PHA copolymer mixture-producing microbial strain (7) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant having the amino acid sequence of SEQ ID NO: 11 (a gene composed of a combination of a part of a PHA synthase gene derived from a microorganism of the genus *Aeromonas* and a part of a PHA synthase gene derived from a microorganism of the genus *Cupriavidus)* are introduced.

### (Microbial Strain Preparation Example)

Preparation of PHA copolymer mixture-producing microbial strain (8)

First, a plasmid for PHA synthase gene introduction was prepared. The preparation was done as follows.

PCR using synthetic oligo DNA was carried out to obtain a DNA fragment (SEQ ID NO: 35) having base sequences upstream and downstream of the B1168 structural gene (whose function is unknown) of *Cupriavidus necator* H16, having a trp promoter, and having a base sequence of a gene encoding a PHA synthase having the amino acid sequence of SEQ ID NO: 11. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1168U-trp-AmNSRe12A506M-B1168D for PHA synthase gene introduction was prepared.

Next, the plasmid vector pNS2X-sacB+B1168U-trp-AmNSRe12A506M-B1168D for PHA synthase gene introduction was introduced into the KNK005dZ/dNSDG/trc-J4b/bktB::lacN19-phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 11 were introduced at a site where the B1168 gene was originally present. The strain thus obtained was named "KNK005dZ/dNSDG/trc-J4b/bktB::lacN19-phaCamSGLVNE/B1168::trp-AmNSRe12A506M" (hereinafter also referred to as "PHA copolymer mixture-producing microbial strain (8)").

The PHA copolymer mixture-producing microbial strain (8) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant having the amino acid sequence of SEQ ID NO: 11 (a gene composed of a combination of a part of a PHA synthase gene derived from a microorganism of the genus *Aeromonas* and a part of a PHA synthase gene derived from a microorganism of the genus *Cupriavidus)* are introduced.

### (Microbial Strain Preparation Example)

Preparation of PHA copolymer mixture-producing microbial strain (9)

First, a plasmid for PHA synthase gene expression was prepared. The preparation was done as follows.

PCR using synthetic oligo DNA was carried out to obtain a DNA fragment (SEQ ID NO: 36) having a base sequence of a gene encoding a PHA synthase having the amino acid sequence of SEQ ID NO: 10. The DNA fragment was digested with restriction enzymes MunI and SpeI, and the resulting DNA fragment was jointed to a plasmid vector pCUP2 which is described in WO 2007/049716 and which was cleaved with MunI and SpeI. In this manner, pCUP2-AmNSRe12 was obtained. Next, PCR using synthetic oligo DNA was carried out to obtain a DNA fragment (SEQ ID NO: 37) having a trp promoter. The DNA fragment was digested with a restriction enzyme MunI, and the resulting DNA fragment was jointed to the pCUP2-AmNSRe12 cleaved with MunI. A conjugate was selected in which the DNA fragment was joined to the pCUP2-AmNSRe12 in such an orientation that the gene encoding a PHA synthase having the amino acid sequence of SEQ ID NO: 10 was located downstream of the trp promoter. In this manner, pCUP2-trp-AmNSRe12 was obtained.

Next, the plasmid pCUP2-trp-AmNSRe12 for PHA synthase gene expression was introduced into the KNK005dZ/dNSDG/trc-J4b/bktB::lacN19-phaCamSGLVNE by electroporation as described above. The resulting strain was named "KNK005dZ/dNSDG/trc-J4b/bktB::lacN19-phaCamSGLVNE/pCUP2-trp-AmNSRe12" (hereinafter also referred to as "PHA copolymer mixture-producing microbial strain (9)").

The PHA copolymer mixture-producing microbial strain (9) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant having the amino acid sequence of SEQ ID NO: 10 (a gene composed of a combination of a part of a PHA synthase gene derived from a microorganism of the genus *Aeromonas* and a part of a PHA synthase gene derived from a microorganism of the genus *Cupriavidus)* are introduced.

### (Microbial Strain Preparation Example)

Preparation of PHA copolymer mixture-producing microbial strain (10)

First, a PHA synthase gene-introduced strain was prepared using the plasmid vector pNS2X-sacB+bktBU-phaCamSGLVNE-bktBD for PHA synthase gene introduction. The preparation was done as follows.

The plasmid vector pNS2X-sacB+bktBU-phaCamSGLVNE-bktBD for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the bktB gene on the chromosome was deleted and in which a gene encoding a PHA synthase having the amino acid sequence of SEQ ID NO: 2 was introduced at a site where the bktB gene was originally present. The strain thus obtained was named "KNK005dZ/dNSDG/trc-J4b/bktB::phaCamSGLVNE".

Next, a plasmid for PHA synthase gene expression was prepared. The preparation was done as follows.

PCR using synthetic oligo DNA was carried out to obtain a DNA fragment (SEQ ID NO: 38) having a base sequence of a gene encoding a PHA synthase having the amino acid sequence of SEQ ID NO: 11. The DNA fragment was digested with restriction enzymes MunI and SpeI, and the resulting DNA fragment was jointed to pCUP2-lacUV5 cleaved with MunI and SpeI. In this manner, a plasmid pCUP2-lacUV5-AmNSRe12A506M for PHA synthase gene expression was obtained.

Next, the plasmid pCUP2-lacUV5-AmNSRe12A506M for PHA synthase gene expression was introduced into the KNK005dZ/dNSDG/trc-J4b/bktB::phaCamSGLVNE by electroporation as described above. The resulting strain was named "KNK005dZ/dNSDG/trc-J4b/bktB::phaCamSGLVNE/pCUP2-lacUV5-AmNSRe12A506M" (hereinafter also referred to as "PHA copolymer mixture-producing microbial strain (10)").

The PHA copolymer mixture-producing microbial strain (10) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant having the amino acid sequence of SEQ ID NO: 11 (a gene composed of a combination of a part of a PHA synthase gene derived from a microorganism of the genus *Aeromonas* and a part of a PHA synthase gene derived from a microorganism of the genus *Cupriavidus)* are introduced.

### (Microbial Strain Preparation Example)

Preparation of PHA copolymer mixture-producing microbial strain (11)

First, a plasmid for PHA synthase gene introduction was prepared. The preparation was done as follows.

PCR using synthetic oligo DNA was carried out to obtain a DNA fragment (SEQ ID NO: 39) having base sequences upstream and downstream of the bktB structural gene (β-ketothiolase enzyme gene) of *Cupriavidus necator* H16, having a lacN19 promoter, and having a base sequence of a gene encoding a PHA synthase having the amino acid sequence of SEQ ID NO: 3. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-lacN19-phaCamSGLVNES389G-bktBD for PHA synthase gene introduction was prepared.

Next, a PHA synthase gene-introduced strain was prepared using the plasmid vector pNS2X-sacB+bktBU-lacN19-phaCamSGLVNES389G-bktBD for PHA synthase gene introduction. The preparation was done as follows.

The plasmid vector pNS2X-sacB+bktBU-lacN19-phaCamSGLVNES389G-bktBD for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the bktB gene on the chromosome was deleted and in which the lacN19 promoter and the gene encoding a PHA synthase having the amino acid sequence of SEQ ID NO: 3 were introduced at a site where the bktB gene was originally present. The strain thus obtained was named "KNK005dZ/dNSDG/trc-J4b/bktB::lacN19-phaCamSGLVNES389G".

Furthermore, the plasmid pCUP2-lacUV5-AmNSRe12A506M for PHA synthase gene expression was introduced into the KNK005dZ/dNSDG/trc-J4b/bktB::lacN19-phaCamSGLVNES389G by electroporation as described above. The resulting strain was named "KNK005dZ/dNSDG/trc-J4b/bktB::lacN19-phaCamSGLVNES389G/pCUP2-lacUV5-AmNSRe12A506M" (hereinafter also referred to as "PHA copolymer mixture-producing microbial strain (11)").

The PHA copolymer mixture-producing microbial strain (11) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 3 and a gene encoding a PHA synthase mutant having the amino acid sequence of SEQ ID NO: 11 (a gene composed of a combination of a part of a PHA synthase gene derived from a microorganism of the genus *Aeromonas* and a part of a PHA synthase gene derived from a microorganism of the genus *Cupriavidus)* are introduced.

### (Microbial Strain Preparation Example)

Preparation of PHA copolymer mixture-producing microbial strain (12)

First, a plasmid for PHA synthase gene introduction was prepared. The preparation was done as follows.

PCR using synthetic oligo DNA was carried out to obtain a DNA fragment (SEQ ID NO: 40) having base sequences upstream and downstream of the bktB structural gene (β-ketothiolase enzyme gene) of *Cupriavidus necator* H16 and having a base sequence of a gene encoding a PHA synthase having the amino acid sequence of SEQ ID NO: 4. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-phaCamSGLVNES389P-bktBD for PHA synthase gene introduction was prepared.

Next, a PHA synthase gene-introduced strain was prepared using the plasmid vector pNS2X-sacB+bktBU-phaCamSGLVNES389P-bktBD for PHA synthase gene introduction. The preparation was done as follows.

The plasmid vector pNS2X-sacB+bktBU-phaCamSGLVNES389P-bktBD for PHA synthase gene introduction was introduced into KNK005dZ/trc-J4b/dbktB/dA1528 by procedures using conjugal transfer as described above.

The KNK005dZ/trc-J4b/dbktB/dA1528 is a strain in which: the phaZ1, phaZ2, and phaZ6 genes on the chromosome of *Cupriavidus necator* H16 are deleted; the PHA synthase gene on the chromosome is substituted with an altered PHA synthase gene derived from the genus *Aeromonas* (a gene encoding a PHA synthase having the amino acid sequence of SEQ ID NO: 2, i.e., N149S/D171G mutant ((NSDG) gene); the expression of the (R)-specific enoyl-CoA hydratase gene on the chromosome is enhanced; and the bktB and A1528 structural genes are deleted. This strain can be prepared according to the method described in WO 2019/142845.

The subsequent culture and selection on Nutrient Agar containing 15% sucrose were carried out as described above to isolate one strain in which the bktB gene on the chromosome was deleted and the gene encoding a PHA synthase having the amino acid sequence of SEQ ID NO: 4 was introduced at a site where the bktB gene was originally present. The strain thus obtained was named "KNK005dZ/dNSDG/trc-J4b/dA1528/bktB::phaCamSGLVNES389P".

Next, a plasmid for PHA synthase gene expression was prepared. The preparation was done as follows.

PCR using synthetic oligo DNA was carried out to obtain a DNA fragment (SEQ ID NO: 41) having a base sequence of a gene encoding a PHA synthase having the amino acid sequence of SEQ ID NO: 13. The DNA fragment was digested with restriction enzymes EcoRI and SpeI, and the resulting DNA fragment was joined to pCUP2-lacUV5 cleaved with MunI and SpeI. In this manner, a plasmid pCUP2-lacUV5-phaCcsA479M for PHA synthase gene expression was obtained.

Next, the plasmid pCUP2-lacUV5-phaCcsA479M for PHA synthase gene expression was introduced into the KNK005dZ/dNSDG/trc-J4b/dA1528/bktB::phaCamSGLVNES389P by electroporation as described above. The resulting strain was named "KNK005dZ/dNSDG/trc-J4b/dA1528/bktB::phaCamSGLVNES389P/pCUP2-lacUV5-phaCcsA479M" (hereinafter also referred to as "PHA copolymer mixture-producing microbial strain (12)").

The PHA copolymer mixture-producing microbial strain (12) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the A1528 gene and the bktB gene on the chromosome are deleted; and a gene encoding a PHA synthase mutant derived from the genus *Aeromonas* and having the amino acid sequence of SEQ ID NO: 4 and a gene encoding a PHA synthase mutant derived from the genus *Chromobacterium* and having the amino acid sequence of SEQ ID NO: 13 are introduced.

### (Microbial Strain Preparation Example)

Preparation of PHA copolymer mixture-producing microbial strain (13)

First, a plasmid for PHA synthase gene introduction was prepared. The preparation was done as follows.

PCR using synthetic oligo DNA was carried out to obtain a DNA fragment (SEQ ID NO: 42) having base sequences upstream and downstream of the B1168 structural gene (whose function is unknown) of *Cupriavidus necator* H16, having a trp promoter, and having a base sequence of a gene encoding a PHA synthase having the amino acid sequence of SEQ ID NO: 9. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1168U-trp-phaCbp-B1168D for PHA synthase gene introduction was prepared.

Next, the plasmid vector pNS2X-sacB+B1168U-trp-phaCbp-B1168D for PHA synthase gene introduction was introduced into the KNK005dZ/dNSDG/trc-J4b/bktB::phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 9 were introduced at a site where the B1168 gene was originally present. The strain thus obtained was named "KNK005dZ/dNSDG/trc-J4b/bktB::phaCamSGLVNE/B1168::trp-phaCbp" (hereinafter also referred to as "PHA copolymer mixture-producing microbial strain (13)").

The PHA copolymer mixture-producing microbial strain (13) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase derived from a soil metagenome and having the amino acid sequence of SEQ ID NO: 9 are introduced.

### (Microbial Strain Preparation Example)

Preparation of PHA copolymer mixture-producing microbial strain (14)

The plasmid vector pNS2X-sacB+B1168U-trp-phaCbp-B1168D for PHA synthase gene introduction was introduced into the KNK005dZ/dNSDG/trc-J4b/bktB::lacN19-phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 9 were introduced at a site where the B1168 gene was originally present. The strain thus obtained was named "KNK005dZ/dNSDG/trc-J4b/bktB::lacN19-phaCamSGLVNE/B1168: :trp-phaCbp" (hereinafter also referred to as "PHA copolymer mixture-producing microbial strain (14)").

The PHA copolymer mixture-producing microbial strain (14) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase derived from a soil metagenome and having the amino acid sequence of SEQ ID NO: 9 are introduced.

### (Microbial Strain Preparation Example)

Preparation of PHA copolymer mixture-producing microbial strain (15)

First, a plasmid for PHA synthase gene expression was prepared. The preparation was done as follows.

PCR using synthetic oligo DNA was carried out to obtain a DNA fragment (SEQ ID NO: 43) having a base sequence of a gene encoding a PHA synthase having the amino acid sequence of SEQ ID NO: 9. The DNA fragment was digested with restriction enzymes MunI and SpeI, and the resulting DNA fragment was joined to pCUP2-lacUV5 cleaved with MunI and SpeI. In this manner, a plasmid pCUP2-lacUV5-phaCbp for PHA synthase gene expression was obtained.

Next, the plasmid pCUP2-lacUV5-phaCbp for PHA synthase gene expression was introduced into the KNK005dZ/dNSDG/trc-J4b/bktB::phaCamSGLVNE by electroporation as described above. The resulting strain was named "KNK005dZ/dNSDG/trc-J4b/bktB::phaCamSGLVNE/pCUP2-lacUV5-phaCbp" (hereinafter also referred to as "PHA copolymer mixture-producing microbial strain (15)").

The PHA copolymer mixture-producing microbial strain (15) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase derived from a soil metagenome and having the amino acid sequence of SEQ ID NO: 9 are introduced.

### (Microbial Strain Preparation Example)

Preparation of PHA copolymer mixture-producing microbial strain (16)

The plasmid pCUP2-lacUV5-phaCbp for PHA synthase gene expression was introduced into the KNK005dZ/dNSDG/trc-J4b/bktB::lacN19-phaCamSGLVNE by electroporation as described above. The resulting strain was named "KNK005dZ/dNSDG/trc-J4b/bktB::lacN19-phaCamSGLVNE/pCUP2-lacUV5-phaCbp" (hereinafter also referred to as "PHA copolymer mixture-producing microbial strain (16)").

The PHA copolymer mixture-producing microbial strain (16) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase derived from a soil metagenome and having the amino acid sequence of SEQ ID NO: 9 are introduced.

### (Microbial Strain Preparation Example)

Preparation of PHA copolymer mixture-producing microbial strain (17)

The plasmid pCUP2-lacUV5-phaCbp for PHA synthase gene expression was introduced into the KNK005dZ/dNSDG/trc-J4b/B1168::trp-phaCesA479W by electroporation as described above. The resulting strain was named "KNK005dZ/dNSDG/trc-J4b/B1168::trp-phaCcsA479W/pCUP2-lacUV5-phaCbp" (hereinafter also referred to as "PHA copolymer mixture-producing microbial strain (17)").

The PHA copolymer mixture-producing microbial strain (17) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; and a gene encoding a PHA synthase mutant derived from the genus *Chromobacterium* and having the amino acid sequence of SEQ ID NO: 12 and a gene encoding a PHA synthase derived from a soil metagenome and having the amino acid sequence of SEQ ID NO: 9 are introduced.

### (Microbial Strain Preparation Example 18)

Preparation of PHA copolymer mixture-producing microbial strain (18)

First, the plasmid vector pNS2X-sacB+B1168U-trp-AmNSRe12A506M-B1168D for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 11 were introduced at a site where the B1168 gene was originally present. The strain thus obtained was named "KNK005dZ/dNSDG/trc-J4b/dbktB/B1168::trp-AmNSRe12A506M".

Next, the plasmid pCUP2-lacUV5-phaCbp for PHA synthase gene expression was introduced into the KNK005dZ/dNSDG/trc-J4b/dbktB/B1168::trp-AmNSRe12A506M by electroporation as described above. The resulting strain was named "KNK005dZ/dNSDG/trc-J4b/dbktB/B1168::trp-AmNSRe12A506M/pCUP2-lacUV5-phaCbp" (hereinafter also referred to as "PHA copolymer mixture-producing microbial strain (18)").

The PHA copolymer mixture-producing microbial strain (18) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant having the amino acid sequence of SEQ ID NO: 11 (a gene composed of a combination of a part of a PHA synthase gene derived from a microorganism of the genus *Aeromonas* and a part of a PHA synthase gene derived from a microorganism of the genus *Cupriavidus*) and a gene encoding a PHA synthase derived from a soil metagenome and having the amino acid sequence of SEQ ID NO: 9 are introduced.

### (Microbial Strain Preparation Example 19)

Preparation of PHA copolymer mixture-producing microbial strain (19)

First, a plasmid for PHA synthase gene expression was prepared. The preparation was done as follows.

PCR using synthetic oligo DNA was carried out to obtain a DNA fragment (SEQ ID NO: 45) having a base sequence of a gene encoding a PHA synthase having the amino acid sequence of SEQ ID NO: 44. The DNA fragment was digested with restriction enzymes MunI and SpeI, and the resulting DNA fragment was joined to pCUP2-lacUV5 cleaved with MunI and SpeI. In this manner, a plasmid pCUP2-lacUV5-phaCbpT357P for PHA synthase gene expression was obtained.

Next, the plasmid pCUP2-lacUV5-phaCbpT357P for PHA synthase gene expression was introduced into the KNK005dZ/dNSDG/trc-J4b/bktB::phaCamSGLVNE by electroporation as described above. The resulting strain was named "KNK005dZ/dNSDG/trc-J4b/bktB::phaCamSGLVNE/pCUP2-lacUV5-phaCbpT357P" (hereinafter also referred to as "PHA copolymer mixture-producing microbial strain (19)").

The PHA copolymer mixture-producing microbial strain (19) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from a soil metagenome and having the amino acid sequence of SEQ ID NO: 44 are introduced.

### (Microbial Strain Preparation Example 20)

Preparation of PHA copolymer mixture-producing microbial strain (20)

First, a plasmid for PHA synthase gene introduction was prepared. The preparation was done as follows.

PCR using synthetic oligo DNA was carried out to obtain a DNA fragment (SEQ ID NO: 46) having base sequences upstream and downstream of the B1168 structural gene (whose function is unknown) of *Cupriavidus necator* H16, having a lacUV5 promoter, and having a base sequence of a gene encoding a PHA synthase having the amino acid sequence of SEQ ID NO: 44. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1168U-lacUV5-phaCbpT357P-B1168D for PHA synthase gene introduction was prepared.

Next, the plasmid vector pNS2X-sacB+B1168U-lacUVS-phaCbpT357P-B1168D for PHA synthase gene introduction was introduced into the KNK005dZ/dNSDG/trc-J4b/bktB::phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the lacUV5 promoter and the gene encoding a PHA synthase having the amino acid sequence of SEQ ID NO: 44 were introduced at a site where the B1168 gene was originally present. The strain thus obtained was named "KNK005dZ/dNSDG/trc-J4b/bktB::phaCamSGLVNE/B1168::lacUV5-phaCbpT357P" (hereinafter also referred to as "PHA copolymer mixture-producing microbial strain (20)").

The PHA copolymer mixture-producing microbial strain (20) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from a soil metagenome and having the amino acid sequence of SEQ ID NO: 44 are introduced.

### (Microbial Strain Preparation Example 21)

Preparation of PHA copolymer mixture-producing microbial strain (21)

First, the plasmid vector pNS2X-sacB+B1168U-lacUV5-phaCbpT357P-B1168D for PHA synthase gene introduction was introduced into the KNK005dZ/dNSDG/trc-J4b/dbktB/PB1185-phaCamSGLVNE by procedures using conjugal transfer as described above. The subsequent culture and selection on Nutrient Agar containing 15% sucrose were carried out as described above to isolate one strain in which the B1168 gene on the chromosome was deleted and in which the lacUV5 promoter and the gene encoding a PHA synthase having the amino acid sequence of SEQ ID NO: 44 were introduced at a site where the B1168 gene was originally present. The strain thus obtained was named "KNK005dZ/dNSDG/trc-J4b/dbktB/PB1185-phaCamSGLVNE/B1168::lacUV5-phaCbpT357P" (hereinafter also referred to as "PHA copolymer mixture-producing microbial strain (21)").

The PHA copolymer mixture-producing microbial strain (21) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from a soil metagenome and having the amino acid sequence of SEQ ID NO: 44 are introduced.

### (Microbial Strain Preparation Example 22)

Preparation of PHA copolymer mixture-producing microbial strain (22)

First, the plasmid vector pNS2X-sacB+B1168U-lacUV5-phaCbpT357P-B1168D for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the B1168 gene on the chromosome was deleted and in which the lacUV5 promoter and the gene encoding a PHA synthase having the amino acid sequence of SEQ ID NO: 44 were introduced at a site where the B1168 gene was originally present. The strain thus obtained was named "KNK005dZ/dNSDG/trc-J4b/dbktB/B1168::lacUV5-phaCbpT357P".

Next, a PHA synthase gene-introduced strain was prepared using the plasmid vector pNS2X-sacB+A2712U-phaCamSGLVNE-A2712 for PHA synthase gene introduction. The preparation was done as follows.

The plasmid vector pNS2X-sacB+A2712U-phaCamSGLVNE-A2712 for PHA synthase gene introduction was introduced into the KNK005dZ/dNSDG/trc-J4b/dbktB/B1168: :lac UV 5-phaCbpT3 57P by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 2 was introduced upstream of the A2712 gene on the chromosome. The strain thus obtained was named "KNK005dZ/dNSDG/trc-J4b/dbktB/B1168::lacUV5-phaCbpT357P/PA2712-phaCamSGLVNE" (hereinafter also referred to as "PHA copolymer mixture-producing microbial strain (22)").

The PHA copolymer mixture-producing microbial strain (22) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 2 and a gene encoding a PHA synthase mutant derived from a soil metagenome and having the amino acid sequence of SEQ ID NO: 44 are introduced.

### (Microbial Strain Preparation Example 23)

Preparation of PHA copolymer mixture-producing microbial strain (23)

First, a plasmid for PHA synthase gene introduction was prepared. The preparation was done as follows.

PCR using synthetic oligo DNA was carried out to obtain a DNA fragment (SEQ ID NO: 47) having base sequences upstream and downstream of the bktB structural gene (β-ketothiolase enzyme gene) of *Cupriavidus necator* H16 and having a base sequence of a gene encoding a PHA synthase having the amino acid sequence of SEQ ID NO: 5. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-phaCamSGLVNES389C-bktBD for PHA synthase gene introduction was prepared.

Next, a PHA synthase gene-introduced strain was prepared using the plasmid vector pNS2X-sacB+bktBU-phaCamSGLVNES389C-bktBD for PHA synthase gene introduction. The preparation was done as follows.

The plasmid vector pNS2X-sacB+bktBU-phaCamSGLVNES389C-bktBD for PHA synthase gene introduction was introduced into the KNK005dZ/dNSDG/trc-J4b/dbktB/B1168: :lac UV 5-phaCbpT3 57P by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 5 was introduced at a site where the bktB gene was originally present on the chromosome. The strain thus obtained was named "KNK005dZ/dNSDG/trc-J4b/dbktB: :phaCamSGLVNES3 89C/B 1168: :lacUV5-phaCbpT357P" (hereinafter also referred to as "PHA copolymer mixture-producing microbial strain (23)").

The PHA copolymer mixture-producing microbial strain (23) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 5 and a gene encoding a PHA synthase mutant derived from a soil metagenome and having the amino acid sequence of SEQ ID NO: 44 are introduced.

### (Microbial Strain Preparation Example 24)

Preparation of PHA copolymer mixture-producing microbial strain (24)

First, a plasmid for PHA synthase gene introduction was prepared. The preparation was done as follows.

PCR using synthetic oligo DNA was carried out to obtain a DNA fragment (SEQ ID NO: 52) having a base sequence upstream of the B1185 structural gene (whose function is unknown) of *Cupriavidus necator* H16, having a part of the base sequence of the B1185 structural gene, and having a base sequence of a gene encoding a PHA synthase having the amino acid sequence of SEQ ID NO: 7. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+B1185U-phaCamSGLVNES389V-B1185D for PHA synthase gene introduction was prepared.

Next, a PHA synthase gene-introduced strain was prepared using the plasmid vector pNS2X-sacB+B1185U-phaCamSGLVNES389V-B1185D for PHA synthase gene introduction. The preparation was done as follows.

The plasmid vector pNS2X-sacB+B1185U-phaCamSGLVNES389V-B1185D for PHA synthase gene introduction was introduced into the KNK005dZ/dNSDG/trc-J4b/dbktB/B1168: :lac UV 5-phaCbpT3 57P by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 7 was introduced at a site where the bktB gene was originally present on the chromosome. The strain thus obtained was named "KNK005dZ/dNSDG/trc-J4b/dbktB/B 1185: :phaCamSGLVNES389V /B 1168: :lacUV5-phaCbpT357P" (hereinafter also referred to as "PHA copolymer mixture-producing microbial strain (24)").

The PHA copolymer mixture-producing microbial strain (24) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 7 and a gene encoding a PHA synthase mutant derived from a soil metagenome and having the amino acid sequence of SEQ ID NO: 44 are introduced.

### (Microbial Strain Preparation Example 25)

Preparation of PHA copolymer mixture-producing microbial strain (25)

First, a plasmid for PHA synthase gene introduction was prepared. The preparation was done as follows.

PCR using synthetic oligo DNA was carried out to obtain a DNA fragment (SEQ ID NO: 49) having base sequences upstream and downstream of the bktB structural gene (β-ketothiolase enzyme gene) of *Cupriavidus necator* H16 and having a base sequence of a gene encoding a PHA synthase having the amino acid sequence of SEQ ID NO: 7. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-phaCamSGLVNES389V-bktBD for PHA synthase gene introduction was prepared.

Next, a PHA synthase gene-introduced strain was prepared using the plasmid vector pNS2X-sacB+bktBU-phaCamSGLVNES389V-bktBD for PHA synthase gene introduction. The preparation was done as follows.

The plasmid vector pNS2X-sacB+bktBU-phaCamSGLVNES389V-bktBD for PHA synthase gene introduction was introduced into the KNK005dZ/dNSDG/trc-J4b/dbktB/B1168: :lac UV 5-phaCbpT3 57P by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 7 was introduced at a site where the bktB gene was originally present on the chromosome. The strain thus obtained was named "KNK005dZ/dNSDG/trc-J4b/dbktB::phaCamSGLVNES389V/B1168::lacUV5-phaCbpT357P" (hereinafter also referred to as "PHA copolymer mixture-producing microbial strain (25)").

The PHA copolymer mixture-producing microbial strain (25) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 7 and a gene encoding a PHA synthase mutant derived from a soil metagenome and having the amino acid sequence of SEQ ID NO: 44 are introduced.

### (Microbial Strain Preparation Example 26)

Preparation of PHA copolymer mixture-producing microbial strain (26)

First, a PHA synthase gene-introduced strain was prepared using the plasmid vector pNS2X-sacB+B1168U-trp-phaCbp-B1168D for PHA synthase gene introduction. The preparation was done as follows.

The plasmid vector pNS2X-sacB+B1168U-trp-phaCbp-B1168D for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the B1168 gene on the chromosome was deleted and in which the trp promoter and the gene encoding a PHA synthase having the amino acid sequence of SEQ ID NO: 9 were introduced at a site where the B1168 gene was originally present. The strain thus obtained was named "KNK005dZ/dNSDG/trc-J4b/dbktB/B1168::trp-phaCbp".

Next, a plasmid for PHA synthase gene introduction was prepared. The preparation was done as follows.

PCR using synthetic oligo DNA was carried out to obtain a DNA fragment (SEQ ID NO: 50) having base sequences upstream and downstream of the bktB structural gene (β-ketothiolase enzyme gene) of *Cupriavidus necator* H16 and having a base sequence of a gene encoding a PHA synthase having the amino acid sequence of SEQ ID NO: 8. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktBU-phaCamSGLVNES389T-bktBD for PHA synthase gene introduction was prepared.

Next, a PHA synthase gene-introduced strain was prepared using the plasmid vector pNS2X-sacB+bktBU-phaCamSGLVNES389T-bktBD for PHA synthase gene introduction. The preparation was done as follows.

The plasmid vector pNS2X-sacB+bktBU-phaCamSGLVNES389T-bktBD for PHA synthase gene introduction was introduced into the KNK005dZ/dNSDG/trc-J4b/dbktB/B1168::trpUV5-phaCbp by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 8 was introduced at a site where the bktB gene was originally present on the chromosome. The strain thus obtained was named "KNK005dZ/dNSDG/trc-J4b/dbktB::phaCamSGLVNES389T/B1168::trp-phaCbp" (hereinafter also referred to as "PHA copolymer mixture-producing microbial strain (26)").

The PHA copolymer mixture-producing microbial strain (26) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 8 and a gene encoding a PHA synthase mutant derived from a soil metagenome and having the amino acid sequence of SEQ ID NO: 9 are introduced.

### (Microbial Strain Preparation Example 27)

Preparation of PHA copolymer mixture-producing microbial strain (27)

A PHA synthase gene-introduced strain was prepared using the plasmid vector pNS2X-sacB+bktBU-phaCamSGLVNES389V-bktBD for PHA synthase gene introduction. The preparation was done as follows.

The plasmid vector pNS2X-sacB+bktBU-phaCamSGLVNES389V-bktBD for PHA synthase gene introduction was introduced into the KNK005dZ/dNSDG/trc-J4b/dbktB/B1168::trp-phaCbp by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 7 was introduced at a site where the bktB gene was originally present on the chromosome. The strain thus obtained was named "KNK005dZ/dNSDG/trc-J4b/dbktB::phaCamSGLVNES389V/B1168::trp-phaCbp" (hereinafter also referred to as "PHA copolymer mixture-producing microbial strain (27)").

The PHA copolymer mixture-producing microbial strain (27) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 7 and a gene encoding a PHA synthase mutant derived from a soil metagenome and having the amino acid sequence of SEQ ID NO: 9 are introduced.

### (Microbial Strain Preparation Example 28)

Preparation of PHA copolymer mixture-producing microbial strain (28)

First, a PHA synthase gene-introduced strain was prepared using the plasmid vector pNS2X-sacB+B1168U-lacUV5-phaCbpT357P-B1168D for PHA synthase gene introduction. The preparation was done as follows.

The plasmid vector pNS2X-sacB+B1168U-lacUV5-phaCbpT357P-B1168D for PHA synthase gene introduction was introduced into the KNK005dZ/dNSDG/trc-J4b/dbktB by procedures using conjugal transfer as described above. The subsequent culture and selection on Nutrient Agar containing 15% sucrose were carried out as described above to isolate one strain in which the B1168 gene on the chromosome was deleted and in which the lacUV5 promoter and the gene encoding a PHA synthase having the amino acid sequence of SEQ ID NO: 44 were introduced at a site where the B1168 gene was originally present. The strain thus obtained was named "KNK005dZ/dNSDG/trc-J4b/dbktB/B1168::lacUV5-phaCbpT357P".

Next, a plasmid for PHA synthase gene introduction was prepared. The preparation was done as follows.

PCR using synthetic oligo DNA was carried out to obtain a DNA fragment (SEQ ID NO: 51) having a base sequence upstream of the A2712 structural gene (whose function is unknown) of *Cupriavidus necator* H16, having a part of the base sequence of the A2712 structural gene, and having a base sequence of a gene encoding a PHA synthase having the amino acid sequence of SEQ ID NO: 7. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+A2712U-phaCamSGLVNES389V-A2712 for PHA synthase gene introduction was prepared.

Next, a PHA synthase gene-introduced strain was prepared using the plasmid vector pNS2X-sacB+A2712U-phaCamSGLVNES389V-A2712 for PHA synthase gene introduction. The preparation was done as follows.

The plasmid vector pNS2X-sacB+A2712U-phaCamSGLVNES389V-A2712 for PHA synthase gene introduction was introduced into the KNK005dZ/dNSDG/trc-J4b/dbktB/B1168: :lac UV 5-phaCbpT3 57P by procedures using conjugal transfer as described above. The subsequent culture and selection on Nutrient Agar containing 15% sucrose were carried out as described above to isolate one strain in which the gene encoding a PHA synthase having the amino acid sequence of SEQ ID NO: 7 was introduced upstream of the A2712 gene on the chromosome. The strain thus obtained was named "KNK005dZ/dNSDG/trc-J4b/dbktB/B1168::lacUV5-phaCbpT357P/PA2712-phaCamSGLVNES389V" (hereinafter also referred to as "PHA copolymer mixture-producing microbial strain (28)").

The PHA copolymer mixture-producing microbial strain (28) is a strain in which: the phaC1 gene (PHA synthase gene), the phaZ1 gene, the phaZ2 gene, and the phaZ6 gene on the chromosome of *Cupriavidus necator* H16 are deleted; the expression of the (R)-specific enoyl-CoA hydratase gene on the chromosome is enhanced; the bktB gene on the chromosome is deleted; and a gene encoding a PHA synthase mutant derived from the genus *Aeromonas* and having the amino acid sequence of SEQ ID NO: 7 and a gene encoding a PHA synthase mutant derived from a soil metagenome and having the amino acid sequence of SEQ ID NO: 44 are introduced.

**[Table 1]**

| PHA copolymer mixture-producing microbial strain | PHA synthase gene (A) | Means and site of introduction of PHA synthase gene (A) | PHA synthase gene (B) | Means and site of introduction of PHA synthase gene (B) | β-Ketothiolase | β-Ketothiolase | (R)-specific enoyl-CoA hydratase |
|---|---|---|---|---|---|---|---|
| | (for synthesis of PHA fraction (I)) | | (for synthesis of PHA fraction (II)) | | bktB gene | A1528 gene | phaJ4b gene |
| (1) | phaCamSGLVNE | A2712 site on genome Without promoter | phaCcsA479W | B1168 site on genome trp promoter | Retained | Retained | Enhanced |
| (2) | phaCamSGLVNE | A2712 site on genome Without promoter | phaCcsA479W | B1168 site on genome trp promoter | Disrupted | Retained | Enhanced |
| (3) | phaCamSGLVNE | bktB site on genome Without promoter | phaCcsA479W | B1168 site on genome trp promoter | Disrupted | Retained | Enhanced |
| (4) | phaCamSGLVNE | A2712 site on genome lac promoter | phaCcsA479W | B1168 site on genome trp promoter | Retained | Retained | Enhanced |
| (5) | phaCamSGLVNE | A2712 site on genome Without promoter | phaCcsA479W | B1185 site on genome trp promoter | Disrupted | Retained | Enhanced |
| (6) | phaCamSGLVNE | B1185 site on genome Without promoter | phaCcsA479W | Plasmid lacUV5 promoter | Disrupted | Retained | Enhanced |
| (7) | phaCamSGLVNE | bktB site on genome lacN19 promoter | AmNSRe12A506M | B1168 site on genome Without promoter | Disrupted | Retained | Enhanced |
| (8) | phaCamSGLVNE | bktB site on genome lacN19 promoter | AmNSRe12A506M | B1168 site on genome trp promoter | Disrupted | Retained | Enhanced |
| (9) | phaCamSGLVNE | bktB site on genome lacN19 promoter | AmNSRe12 | Plasmid trp promoter | Disrupted | Retained | Enhanced |
| (10) | phaCamSGLVNE | bktB site on genome Without promoter | AmNSRe12A506M | Plasmid lacUV5 promoter | Disrupted | Retained | Enhanced |
| (11) | phaCamSGLVNES389G | bktB site on genome lacN19 promoter | AmNSRe12A506M | Plasmid lacUV5 promoter | Disrupted | Retained | Enhanced |
| (12) | phaCamSGLVNES389P | bktB site on genome Without promoter | phaCcsA479M | Plasmid lacUV5 promoter | Disrupted | Disrupted | Enhanced |
| (13) | phaCamSGLVNE | bktB site on genome Without promoter | phaCbp | B1168 site on genome trp promoter | Disrupted | Retained | Enhanced |
| (14) | phaCamSGLVNE | bktB site on genome lacN19 promoter | phaCbp | B1168 site on genome trp promoter | Disrupted | Retained | Enhanced |

**[Table 2]**

| PHA copolymer mixture-producing microbial strain | PHA synthase gene (A) | Means and site of introduction of PHA synthase gene (A) | PHA synthase gene (B) | Means and site of introduction of PHA synthase gene (B) | β-Ketothiolase | β-Ketothiolase | (R)-specific enoyl-CoA hydratase |
|---|---|---|---|---|---|---|---|
| | (for synthesis of PHA fraction (I)) | | (for synthesis of PHA fraction (II) | | bktB gene | A1528 gene | phaJ4b gene |
| (15) | phaCamSGLVNE | bktB site on genome Without promoter | phaCbp | Plasmid lacUV5 promoter | Disrupted | Retained | Enhanced |
| (16) | phaCamSGLVNE | bktB site on genome lacN19 promoter | phaCbp | Plasmid lacUV5 promoter | Disrupted | Retained | Enhanced |
| (17) | phaCbp | Plasmid lacUV5 promoter | phaCcsA479W | B1168 site on genome trp promoter | Retained | Retained | Enhanced |
| (18) | phaCbp | Plasmid lacUV5 promoter | AmNSRe12A506M | B1168 site on genome trp promoter | Disrupted | Retained | Enhanced |
| (19) | phaCamSGLVNE | bktB site on genome Without promoter | phaCbpT357P | Plasmid lacUV5 promoter | Disrupted | Retained | Enhanced |
| (20) | phaCamSGLVNE | bktB site on genome Without promoter | phaCbpT357P | B1168 site on genome lacUV5 promoter | Disrupted | Retained | Enhanced |
| (21) | phaCamSGLVNE | B1185 site on genome Without promoter | phaCbpT357P | B1168 site on genome lacUV5 promoter | Disrupted | Retained | Enhanced |
| (22) | phaCamSGLVNE | A2712 site on genome Without promoter | phaCbpT357P | B1168 site on genome lacUV5 promoter | Disrupted | Retained | Enhanced |
| (23) | phaCamSGLVNE S389C | bktB site on genome Without promoter | phaCbpT357P | B1168 site on genome lacUV5 promoter | Disrupted | Retained | Enhanced |
| (24) | phaCamSGLVNE S389V | B1185 site on genome Without promoter | phaCbpT357P | B1168 site on genome lacUV5 promoter | Disrupted | Retained | Enhanced |
| (25) | phaCamSGLVNE S389V | bktB site on genome Without promoter | phaCbpT357P | B1168 site on genome lacUV5 promoter | Disrupted | Retained | Enhanced |
| (26) | phaCamSGLVNE S389T | bktB site on genome Without promoter | phaCbp | B1168 site on genome trp promoter | Disrupted | Retained | Enhanced |
| (27) | phaCamSGLVNE S389V | bktB site on genome Without promoter | phaCbp | B1168 site on genome trp promoter | Disrupted | Retained | Enhanced |
| (28) | phaCamSGLVNE S389V | A2712 site on genome Without promoter | phaCbpT357P | B1168 site on genome lacUV5 promoter | Disrupted | Retained | Enhanced |

### (Example 1)

PHA production by PHA copolymer mixture-producing microbial strain (1)

Culture examination using the PHA copolymer mixture-producing microbial strain (1) was conducted under the conditions described below.

### (Culture Media)

The seed culture medium was composed of 1 w/v% Meat-extract, 1 w/v% Bacto-Tryptone, 0.2 w/v% Yeast-extract, 0.9 w/v% Na₂HPO₄•12H₂O, and 0.15 w/v% KH₂PO₄ (pH = 6.8).

The preculture medium was composed of 1.1 w/v% Na₂HPO₄•12H₂O, 0.19 w/v% KH₂PO₄, 1.29 w/v% (NH₄)₂SO₄, 0.1 w/v% MgSO₄•7H₂O, 2.5 w/v% palm olein oil, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1 N hydrochloric acid).

The PHA production culture medium was composed of 0.385 w/v% Na₂HPO₄•12H₂O, 0.067 w/v% KH₂PO₄, 0.291 w/v% (NH₄)₂SO₄, 0.1 w/v% MgSO₄•7H₂O, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1 N hydrochloric acid).

### (Method for Measuring Percentage of Accumulated PHA)

The percentage of accumulated PHA to dried microbial cells was measured as follows. The microbial cells were collected from the culture fluid by centrifugation. The collected microbial cells were washed with ethanol and freeze-dried to give dried microbial cells, the weight of which was measured. To 1 g of the dried microbial cells was added 100 ml of chloroform, and the microbial cells in chloroform were stirred at room temperature for a day to extract a PHA (PHA copolymer mixture) from the microbial cells. The residues of the microbial cells were removed by filtration, and the filtrate was concentrated using an evaporator to a total volume of 30 ml. To the concentrate was slowly added 90 ml of hexane, and the liquid mixture was left for 1 hour under gentle stirring. The PHA precipitated was collected by filtration and vacuum-dried at 50°C for 3 hours. The weight of the dried PHA was measured, and the percentage of the accumulated PHA to the dried microbial cells was calculated.

### (Assay of PHA Copolymer)

The "PHA fraction (I)" and the "PHA composed mainly of the PHA fraction (II)" in the PHA copolymer mixture were separately collected by the MIBK fractionation previously described.

The average 3HH content of each of the PHA copolymer mixture and the PHA fraction (I) was assayed by the previously-described average 3HH content measurement method using gas chromatography.

The average 3HH content of the PHA fraction (II) was assayed by the previously-described average 3HH content measurement method using a DSC.

The weight percentage of each of the PHA fractions (I) and (II) in the PHA copolymer mixture was calculated by the previously-described method for calculating the weight percentage of each fraction in the PHA copolymer mixture.

### (PHA Production Culture)

PHA production culture was performed as follows. First, a glycerol stock (50 µl) of the PHA copolymer mixture-producing microbial strain (1) was inoculated into the seed culture medium (10 ml) and cultured for 24 hours to accomplish seed culture. Next, the seed culture fluid was inoculated at a concentration of 1.0 v/v% into a 3-L jar fermenter (MDL-300, manufactured by B.E. Marubishi Co., Ltd.) charged with 1.8 L of the preculture medium. The fermenter was operated at a culture temperature of 30°C, a stirring speed of 500 rpm, and an aeration of 1.8 L/min, and the preculture was conducted for 28 hours during which the pH was controlled between 6.7 and 6.8. For the pH control, a 14% aqueous solution of ammonium hydroxide was used.

Next, the preculture fluid was inoculated at a concentration of 5.0 v/v% into a 5-L jar fermenter (MDS-U50, manufactured by B.E. Marubishi Co., Ltd.) charged with 2.5 L of the PHA production culture medium. The fermenter was operated at a culture temperature of 33°C, a stirring speed of 420 rpm, and an aeration of 2.1 L/min, and the pH was controlled between 6.7 and 6.8. For the pH control, a 25% aqueous solution of ammonium hydroxide was used. The carbon source was added intermittently. Palm olein oil was used as the carbon source. The culture was continued until the percentage of accumulated PHA to dried microbial cells reached 80% or more. The percentage of accumulated PHA to dried microbial cells, the average 3HH contents of the PHA copolymer mixture, PHA fraction (I), and PHA fraction (II), and the weight percentages of the PHA fractions (I) and (II) were measured as described above. The results are listed in Table 3.

### (Examples 2 to 28)

PHA production by PHA copolymer mixture-producing microbial strains (2) to (28)

Culture examination using the PHA copolymer mixture-producing microbial strains (2) to (28) was conducted under the same conditions as the culture examination in Example 1. The percentage of accumulated PHA to dried microbial cells, the average 3HH contents of the PHA copolymer mixture, PHA fraction (I), and PHA fraction (II), and the weight percentages of the PHA fractions (I) and (II) are listed in Table 3.

**[Table 3]**

| | PHA copolymer mixture-producing microbial strain | Percentage of accumulated PHA | 3HH content in total mixture | Weight percentage of PHA fraction (I) | 3HH content in PHA fraction (I) | Weight percentage of PHA fraction (II) | 3HH content in PHA fraction (II) |
|---|---|---|---|---|---|---|---|
| | | (wt%) | (mol%) | (%) | (mol%) | (%) | (mol%) |
| Ex. 1 | (1) | 90 | 1.6 | 12.4 | 13.5 | 87.6 | 0.0 |
| Ex. 2 | (2) | 90 | 2.6 | 15.6 | 17.6 | 84.4 | 0.0 |
| Ex. 3 | (3) | 90 | 3.4 | 20.0 | 18.0 | 80.0 | 0.0 |
| Ex. 4 | (4) | 91 | 3.9 | 32.5 | 12.2 | 67.5 | 0.0 |
| Ex. 5 | (5) | 89 | 2.8 | 19.2 | 15.0 | 80.8 | 0.0 |
| Ex. 6 | (6) | 90 | 1.9 | 11.0 | 17.9 | 89.0 | 0.0 |
| Ex. 7 | (7) | 89 | 6.4 | 43.5 | 12.4 | 56.5 | 1.9 |
| Ex. 8 | (8) | 87 | 4.8 | 15.8 | 17.3 | 84.2 | 2.5 |
| Ex. 9 | (9) | 86 | 4.6 | 17.1 | 19.6 | 82.9 | 1.7 |
| Ex. 10 | (10) | 88 | 4.4 | 14.7 | 16.7 | 85.3 | 2.4 |
| Ex. 11 | (11) | 87 | 5.9 | 32.6 | 14.3 | 67.4 | 2.0 |
| Ex. 12 | (12) | 86 | 6.2 | 28.9 | 11.9 | 71.1 | 4.0 |
| Ex. 13 | (13) | 89 | 6.7 | 16.0 | 11.1 | 84.0 | 5.9 |
| Ex. 14 | (14) | 89 | 6.8 | 19.0 | 12.3 | 81.0 | 5.5 |
| Ex. 15 | (15) | 87 | 7.6 | 27.3 | 13.0 | 72.7 | 5.6 |
| Ex. 16 | (16) | 89 | 6.5 | 21.3 | 10.6 | 78.7 | 5.4 |
| Ex. 17 | (17) | 89 | 2.6 | 28.0 | 9.4 | 72.0 | 0.0 |
| Ex. 18 | (18) | 87 | 3.6 | 6.0 | 19.9 | 94.0 | 2.6 |
| Ex. 19 | (19) | 89 | 5.1 | 17.1 | 12.3 | 82.9 | 3.7 |
| Ex. 20 | (20) | 87 | 5.2 | 14.1 | 10.8 | 85.9 | 4.3 |
| Ex. 21 | (21) | 87 | 4.7 | 9.4 | 11.0 | 90.6 | 4.1 |
| Ex. 22 | (22) | 87 | 4.6 | 1.7 | 13.6 | 98.3 | 4.5 |
| Ex. 23 | (23) | 89 | 6.0 | 22.3 | 12.7 | 77.7 | 4.2 |
| Ex. 24 | (24) | 88 | 4.4 | 8.0 | 13.1 | 92.0 | 3.7 |
| Ex. 25 | (25) | 89 | 6.8 | 28.0 | 12.9 | 72.0 | 4.5 |
| Ex. 26 | (26) | 91 | 9.7 | 53.0 | 13.1 | 47.0 | 6.0 |
| Ex. 27 | (27) | 86 | 6.7 | 25.0 | 14.5 | 75.0 | 4.2 |
| Ex. 28 | (28) | 89 | 5.4 | 15.6 | 12.8 | 84.4 | 4.1 |

Table 3 reveals that in each of Examples 1 to 28, the microbial culture successfully yielded a PHA copolymer mixture which contained a PHA fraction (I) having an average 3HH content of 9 to less than 20 mol% and a PHA fraction (II) having an average 3HH content of 0 to 8 mol% and in which the weight percentage of the PHA fraction (II) was 45% or more.

### (Reference Example 1)

Evaluation of granulation properties of PHA produced by PHA copolymer mixture-producing microbial strain (4)

The PHA obtained by PHA production culture of the PHA copolymer mixture-producing microbial strain (4) was subjected to spray drying and evaluated for granulation properties. The spray drying and the evaluation were conducted as described below.

### (Spray Drying)

The culture fluid of the PHA copolymer mixture-producing microbial strain (4) was treated by procedures as described in paragraphs [0085] to [0087] of WO 2021/251049 to obtain an aqueous PHA suspension.

The obtained aqueous PHA suspension was adjusted to a concentration of 50 wt%, and 1.0 phr (1 part by weight per 100 parts by weight of the PHA in the aqueous suspension) of a non-ionic ethylene oxide/propylene oxide copolymer dispersant (polyethylene oxide molecular weight = 8,000, polypropylene oxide molecular weight = 2,000, trade name: PLONON 208) was added and mixed with the aqueous suspension to give a liquid mixture. The liquid mixture was then stirred for 120 minutes, after which 0.4 ml of 10 wt% sulfuric acid was added per 100 g of the liquid mixture to obtain an aqueous PHA suspension having a pH of 3.0. The obtained aqueous PHA suspension was subjected to spray drying using a spray dryer (OC-16) manufactured by Ohkawara Kakohki Co., Ltd. (hot air temperature = 160°C, discharged air temperature = 105°C, rotary atomizer rotational speed = 13,000 rpm). Thus, a PHA powder was obtained.

### (Evaluation of Granulation Properties)

The evaluation of the granulation properties was conducted as follows using the amount of fine powder particles as an index of the granulation properties.

An amount of 0.05 g of sodium dodecyl sulfate, which is a surfactant, was added as a dispersant to 20 ml of ion-exchanged water, and thus an aqueous surfactant solution was obtained. After that, 0.2 g of the PHA powder was added and dispersed in the aqueous surfactant solution to prepare a dispersion as a measurement object. The prepared dispersion was introduced into a laser diffraction/scattering particle size distribution analyzer (LA-950 of Horiba, Ltd.), and the particle size of the powder particles was measured. The amount of fine powder particles with a particle size of 20 µm or less in the PHA powder is shown in Table 4.

### (Reference Examples 2 and 3)

### Evaluation of granulation properties of PHAs produced by PHA copolymer mixture-producing microbial strains (5) and (8)

The PHAs produced by PHA production culture of the PHA copolymer mixture-producing microbial strains (5) and (8) were subjected to spray drying and evaluated for granulation properties. The spray drying and the evaluation were conducted under the same conditions as in Reference Example 1. The amount of fine powder particles with a particle size of 20 µm or less in the PHA powder is shown in Table 4.

### (Reference Examples 4 and 5)

### Evaluation of granulation properties of PHAs produced by PHA copolymer mixture-producing microbial strains (13) and (15)

The PHAs produced by PHA production culture of the PHA copolymer mixture-producing microbial strains (13) and (15) were subjected to spray drying and evaluated for granulation properties. The spray drying and the evaluation were conducted under the same conditions as in Reference Example 1, except that in the spray drying the hot air temperature was changed to 140°C and the discharged air temperature was changed to 90°C. The amount of fine powder particles with a particle size of 20 µm or less in the PHA powder is shown in Table 4.

### (Comparative Example 1)

### Evaluation of granulation properties of P(3BB)

A PHA obtained by PHA production culture of a P(3HB)-producing microbial strain (*Cupriavidus necator* H16) was subjected to spray drying for the purpose of evaluating the granulation properties of the PHA. The spray drying was attempted under the same conditions as in Reference Example 1. However, in this example, almost no granulation was observed, and evaluation was not able to be made due to the risk of dust explosion. The result is shown in Table 4.

### (Comparative Examples 2 and 3)

### Evaluation of granulation properties of P(3HB-co-3HH)

PHAs obtained by PHA production culture of two types of P(3HB-co-3HH)-producing microbial strains (transformed *Cupriavidus necator* strains) were subjected to spray drying and evaluated for granulation properties. The spray drying and the evaluation were conducted under the same conditions as in Reference Example 1. The amount of fine powder particles with a particle size of 20 µm or less in the PHA powder is shown in Table 4. It should be noted that each of the microbial strains has only one type of polyhydroxyalkanoate synthase and does not produce the polyhydroxyalkanoate copolymer mixture of the present invention.

**[Table 4]**

| | PHA copolymer mixture-producing microbial strain | 3HH content in total mixture | Weight percentage of PHA fraction (I) | 3HH content in PHA fraction (I) | Weight percentage of PHA fraction (II) | 3HH content in PHA fraction (II) | Hot air temperature | Discharged air temperature | ATM rotational speed | Amount of fine particles | Evaluation of granulation properties |
|---|---|---|---|---|---|---|---|---|---|---|---|
| | | (mol%) | (%) | (mol%) | (%) | (mol%) | (°C) | (°C) | (rpm) | [vol%] | |
| Ref. 1 | (4) | 3.9 | 32.5 | 12.2 | 67.5 | 0.0 | 160 | 105 | 13000 | 17.0 | Good |
| Ref. 2 | (5) | 2.8 | 19.2 | 15.0 | 80.8 | 0.0 | 160 | 105 | 13000 | 7.0 | Good |
| Ref. 3 | (8) | 4.8 | 15.8 | 17.3 | 84.2 | 2.5 | 160 | 105 | 13000 | 10.0 | Good |
| Ref. 4 | (13) | 6.7 | 16.0 | 11.1 | 84.0 | 5.9 | 140 | 90 | 13000 | 27.0 | Good |
| Ref. 5 | (15) | 7.6 | 27.3 | 13.0 | 72.7 | 5.6 | 140 | 90 | 13000 | 6.0 | Good |
| Comp. 1 | - | 0.0 | - | - | 100 | 0.0 | - | - | - | - | Evaluation was impossible |
| Comp. 2 | - | 3.4 | - | - | 100 | 3.4 | 160 | 105 | 13000 | 59.2 | Poor |
| Comp. 3 | - | 5.0 | - | - | 100 | 5.0 | 140 | 90 | 13000 | 44.7 | Poor |

Table 4 shows the amount of the fine powder particles was much smaller in Reference Examples 1 to 5 than in Comparative Examples 1 to 3 where the drying conditions were the same as those in Reference Examples 1 to 3 or 4 and 5.

## Claims

1. A method for producing a polyhydroxyalkanoate copolymer mixture, the method comprising the step of culturing a microorganism that produces the polyhydroxyalkanoate copolymer mixture, wherein
the polyhydroxyalkanoate copolymer mixture contains:
a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.

2. The method according to claim 1, wherein an average 3-hydroxyhexanoate content in the total polyhydroxyalkanoate copolymer mixture is from 0.5 to 14 mol%.

3. The method according to claim 1 or 2, wherein the microorganism has genes encoding two types of polyhydroxyalkanoate synthases differing in polymerization activity for (R)-3-hydroxyhexanoyl-CoA.

4. The method according to claim 3, wherein an amino acid sequence identity between the two types of polyhydroxyalkanoate synthases differing in polymerization activity for (R)-3-hydroxyhexanoyl-CoA is 90% or less.

5. The method according to claim 3, wherein the genes encoding the two types of polyhydroxyalkanoate synthases differing in polymerization activity for (R)-3-hydroxyhexanoyl-CoA include:
a gene (A) encoding a polyhydroxyalkanoate synthase that synthesizes a polyhydroxyalkanoate copolymer having 3-hydroxybutyrate structural units and 3-hydroxyhexanoate structural units; and
a gene (B) encoding a polyhydroxyalkanoate synthase having lower polymerization activity for (R)-3-hydroxyhexanoyl-CoA than the polyhydroxyalkanoate synthase encoded by the gene (A).

6. The method according to claim 5, wherein in the microorganism, a level of expression of the gene (A) is regulated to be lower than a level of expression of the gene (B).

7. The method according to claim 5, wherein the gene (A) is a polyhydroxyalkanoate synthase gene derived from a microorganism of the genus *Aeromonas* or a mutant of the polyhydroxyalkanoate synthase gene.

8. The method according to claim 7, wherein the gene (A) is a gene encoding an amino acid sequence having a sequence identity of 99.5 to 100% with an amino acid sequence of any one of SEQ ID NOS: 1 to 8.

9. The method according to claim 5, wherein the gene (B) is composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus.*

10. The method according to claim 9, wherein the gene (B) is a gene encoding an amino acid sequence having a sequence identity of 90 to 100% with an amino acid sequence of SEQ ID NO: 10 or 11.

11. The method according to claim 5, wherein the gene (B) is a polyhydroxyalkanoate synthase gene derived from a microorganism of the genus *Chromobacterium* or a mutant of the polyhydroxyalkanoate synthase gene.

12. The method according to claim 11, wherein the gene (B) is a gene encoding an amino acid sequence having a sequence identity of 90 to 100% with an amino acid sequence of SEQ ID NO: 12 or 13.

13. The method according to claim 5, wherein either one of the genes (A) and (B) is a gene encoding an amino acid sequence having a sequence identity of 90 to 100% with an amino acid sequence of SEQ ID NO: 9 or 44.

14. The method according to claim 1 or 2, wherein the microorganism is a microorganism having a gene encoding a protein that exhibits (R)-specific enoyl-CoA hydratase activity.

15. The method according to claim 1 or 2, wherein the microorganism is a transformed microorganism that has been transformed to supply a larger amount of (R)-3-hydroxyhexanoyl-CoA to an intracellular polyhydroxyalkanoate synthase than a wild strain of the microorganism.

16. The method according to claim 15, wherein the transformed microorganism is a microorganism that has been transformed to enhance expression of a gene encoding a protein that exhibits (R)-specific enoyl-CoA hydratase activity.

17. The method according to claim 15, wherein the transformed microorganism is a microorganism that has been transformed to inhibit expression of a gene encoding a β-ketothiolase enzyme having thiolysis activity for β-ketohexanoyl-CoA which is β-ketoacyl-CoA having six carbon atoms.

18. The method according to claim 1 or 2, wherein a carbon source containing an oil or a fatty acid is added in the culturing step.

19. The method according to claim 1 or 2, wherein the microorganism belongs to the genus *Cupriavidus* or is a transformant of a microorganism of the genus *Cupriavidus.*

20. The method according to claim 19, wherein the microorganism is *Cupriavidus necator* or a transformant of *Cupriavidus necator.*

21. The method according to claim 1 or 2, further comprising the steps of:
after culturing the microorganism, disrupting cells of the microorganism, purifying the polyhydroxyalkanoate copolymer mixture, and obtaining an aqueous suspension of the polyhydroxyalkanoate copolymer mixture; and
spray-drying the aqueous suspension to obtain a powder of the polyhydroxyalkanoate copolymer mixture.

22. A transformed microorganism that produces a polyhydroxyalkanoate copolymer mixture, the transformed microorganism comprising genes encoding two types of polyhydroxyalkanoate synthases differing in polymerization activity for (R)-3-hydroxyhexanoyl-CoA, wherein
the polyhydroxyalkanoate copolymer mixture contains:
a polyhydroxyalkanoate fraction (I) that contains a polyhydroxyalkanoate copolymer having 3-hydroxybutyrate structural units and 3-hydroxyhexanoate structural units and that has an average 3-hydroxyhexanoate content of 9 to less than 20 mol%; and
a polyhydroxyalkanoate fraction (II) that contains a polyhydroxyalkanoate having 3-hydroxybutyrate structural units and that has an average 3-hydroxyhexanoate content of 0 to 8 mol%, and
a weight percentage of the polyhydroxyalkanoate fraction (II) in the polyhydroxyalkanoate copolymer mixture is 45% or more.
